# EUROPEAN PATENT SPECIFICATION

(11) **EP 3 930 838 B1**
(45) Date of publication and mention of the grant of the patent: **10.07.2024**
(21) Application number: 20714389.2
(22) Date of filing: 26.02.2020
(51) Int. Cl.: A61P 3/00, A61P 29/00, A61P 31/00, A61P 35/00, C07D 401/12, C07D 401/14, C07D 405/12, C07D 413/12, A61K 31/4427, A61K 31/4433, A61K 31/4439, A61K 31/444, A61K 31/4545

(54) **N-(PYRIDINYL)ACETAMIDE DERIVATIVES AS P300/CBP HAT INHIBITORS AND METHODS FOR THEIR USE**
N-(PYRIDINYL)ACETAMIDDERIVATE ALS P300/CBP-HAT-INHIBITOREN UND VERFAHREN ZU DEREN VERWENDUNG
DÉRIVÉS DE N-(PYRIDINYL)ACÉTAMIDE EN TANT QU'INHIBITEURS DE P300/CBP HAT ET LEURS MÉTHODES D'UTILISATION

(30) Priority: 27.02.2019 US 201962811143 P; 29.07.2019 US 201962879852 P
(43) Date of publication of application: 05.01.2022
(73) Proprietor: Constellation Pharmaceuticals, Inc., Boston, MA 02210 (US)
(72) Inventor: WILSON, Jonathan, E., Arlington, MA 02474 (US)
(74) Representative: Potter Clarkson
(86) International application number: PCT/US2020/019786
(87) International publication number: WO 2020/176558

(56) References cited:
- WO-A1-2016/196117
- WO-A1-2019/161162
- WO-A2-2017/205536
- SARAH E. WINTERTON ET AL: "Discovery of Cytochrome P450 4F11 Activated Inhibitors of Stearoyl Coenzyme A Desaturase", JOURNAL OF MEDICINAL CHEMISTRY, vol. 61, no. 12, 28 June 2018 (2018-06-28), pages 5199 - 5221, XP055698788, ISSN: 0022-2623, DOI: 10.1021/acs.jmedchem.8b00052

## Description

### CROSS-REFERENCE TO RELATED APPLICATIONS

This application claims priority to U.S. Provisional Application No. 62/879,852, filed July 29, 2019 and U.S. Provisional Application No. 62/811,143, filed February 27, 2019.

### BACKGROUND

Chromatin is a complex combination of DNA and protein that makes up chromosomes. It is found inside the nuclei of eukaryotic cells and is divided between heterochromatin (condensed) and euchromatin (extended) forms. The major components of chromatin are DNA and proteins. Histones are the chief protein components of chromatin, acting as spools around which DNA winds. The functions of chromatin are to package DNA into a smaller volume to fit in the cell, to strengthen the DNA to allow mitosis and meiosis, and to serve as a mechanism to control expression and DNA replication. The chromatin structure is controlled by a series of post-translational modifications to histone proteins, notably histones H3 and H4, and most commonly within the "histone tails" which extend beyond the core nucleosome structure. Histone tails tend to be free for protein-protein interaction and are also the portion of the histone most prone to post-translational modification (Goll and Bestor, 2002, Genes Dev. 16:1739- 1742; Grant, 2001, Genome Biol. 2:). These modifications include acetylation, methylation, phosphorylation, ubiquitinylation, SUMOylation. These epigenetic marks are written and erased by specific enzymes that place the tags on specific residues within the histone tail, thereby forming an epigenetic code, which is then interpreted by the cell to allow gene specific regulation of chromatin structure and thereby transcription.

Covalent modification of histones is a fundamental mechanism of control of gene expression, and one of the major epigenetic mechanisms at play in eukaryotic cells (Kouzarides, Cell, 128, 693-705 (2007)). Because distinct transcriptional states define fundamental cellular processes, such as cell type specification, lineage commitment, cell activation and cell death, their aberrant regulation is at the core of a range of diseases (Medzhitov et al., Nat. Rev. Immunol., 9, 692-703 (2009); Portela et al., Nat. Biotech., 28, 1057-1068 (2010)). Distinct classes of enzymes, namely histone acetyltransferases (HATS) and histone deacetylases (HDACs), acetylate or de-acetylate specific histone lysine residues (Struhl K., Genes Dev., 1998, 12, 5, 599-606).

Histone acetyltransferases (HATs) catalyze the acetylation (transfer of an acetyl group) on a ε-amino group of a target lysine side chain within a substrate histone, and histone deacetylases (HDACs) catalyze the removal of acetyl groups from lysine residues. Subsequently, acetylated core histones were shown to preferentially associate with transcriptionally active chromatin. See Nucleic Acids Res. 5:1863-1876 (1978); Proc. Natl. Acad. Sci. 75:2239-2243 (1978); and EMBO J. 7:1395-1402 (1988). HATs are categorized into four major families based on primary sequence homology, shared structural features, and functional roles: Gcn5/PCAF (General control nonrepressed protein 5 and p300 and CBP associated factor); MYST (named for the founding members MOZ, Ybf2/Sas3, Sas2, and Tip60); p300/CBP (protein of 300kDa and CREB Binding Protein); and Rtt109 (Regulator of Tyl Transposition gene production 109).

Paralogs p300 and CBP (CREB binding protein) were originally identified as binding partners of the adenovirus early-region 1A (E1A) protein (Yee and Branton, 1985, Virology 147:142-153; Harlow et al., 1986, Mol. Cell Biol. 6:1579-1589), and the cAMP-regulated enhancer (CRE) binding proteins (Chrivia et al, 1993, Nature 365:855-859), respectively. p300 and CBP HAT domains have >90% sequence identity and are conserved in metazoans with many overlapping functions. In addition to the HAT domain, p300/CBP contains other protein interaction domains including three cysteine-histidine rich domains (CH1, CH2 and CH3), a KIX domain, a bromodomain, and a steroid receptor coactivator interaction domain (SID, also the SRC-1 interaction domain) (Arany et al, Cell. 1994 Jun 17;77(6):799-800) p300/CBP was found to have intrinsic HAT activity (Ogryzko et al., 1996, Cell 87:953-959; Bannister and Kouzarides, 1996, Nature 384:641-643). In addition to acetylating multiple lysines on all four core histones (H2A, H2B, H3 and H4), p300/CBP has been shown to have acetyltransferase activity towards > 70 substrates (Wang et al., 2008, Curr. Opin.Struct. Biol. 18:741-747), including, for example, p53 (Gu et al., 1997, Cell 90:595-606), MyoD (Polesskaya et al., 2002, J. Biol. Chem. 275:34359-64), STAT3 (Yuan et al., 2005, Science 307:269-73) and NFκβ (Chen et al., 2002, EMBO J. 21:6539-48). These two acetyltransferases are responsible for the majority of histone H3 lysine 18 acetylation (H3K18ac) and H3K27ac, modifications associated with active promoters and enhancers (Horwitz et al. 2008; Jin et al. 2011).

Besides acting as an acetyltransferase, p300 also acts as a scaffold for transcription factors or a bridge to connect the transcription factors and the basal transcriptional machinery to activate transcription (Chan and Thangue, 2001, J. Cell Sci. 114:2363-2373; Chen and Li, 2011, Epigenetics 6:957-961). P300/CBP proteins are involved in many cellular processes, including cell growth, proliferation, and differentiation (reviewed in Chan and Thangue, 2001, J. Cell Sci. 114:2363-2373). Mutations in p300/CBP have been observed in number of human diseases, particularly cancer with frequencies up to 30%. A higher frequency of these mutations occur within the HAT domain, suggesting a selective pressure to alter this activity in cancers. These mutations are mostly mono-allelic, with loss of heterozygosity of the second allele, consistent with Knudson's hypothesis of a classical tumor suppressor gene. See Nature 376, 348-351, 1995; Oncogene 12, 1565-1569, 1996; and Proc. Natl. Acad. Sci. USA 94, 8732-8737, 1997. Heterozygous mutations in *CBP* were first described in RTS, an autosomal-dominant disease, characterised by mental retardation, skeletal abnormalities and a high incidence of neoplasia (Nature 376, 348-351, 1995). This suggests that a full complement of CBP gene dosage is required for normal development. P300/CBP genes are also involved in various chromosomal translocations, particularly in hematological malignancies and possibly contribute to aberrant growth through gain of function (Kitabayashi et al. 2001; Panagopoulos et al. 2001)

High p300 expression, correlating with poor survival and aggressive phenotypes, has been observed in prostate cancer (Debes et al 2003; Cancer Res. 63: 7638-7640; Heemers et al., 2008, Adv. Exp. Med. Biol. 617:535-40; Isharwal et al., 2008, Prostate 68:1097-104), liver cancer (Yokomizo et al., 2011, Cancer Lett. 310:1407; Li et al., 2011, J. Transl. Med. 9:5), breast cancer (Fermento et al., 2010, Exp. Mol. Pathol. 88:256-64), esophageal carcinoma (Li et al, 2011, Ann Thorac Surg. 91: 1531-1538) and cutaneous squamous cell carcinoma (Chen et al, 2014, Br J Dermatol. 172: 111-119). Inhibition of p300/CBP has therapeutic potential in cancer (Iyer et al., 2004, Proc. Natl. Acad. Sci. USA 101:7386-7391; Stimson et al., 2005, Mol. Cancer Ther. 4:1521-1532; Zheng et al., 2004, Methods Enzymol. 376:188-199), cardiac disease (Davidson et al., 2005, Chembiochem. 6:162-170); diabetes mellitus (Zhou et al., 2004, Nat. Med. 10:633-637), and HIV (Varier and Kundu, 2006, Curr. Pharm. Des. 12:1975-1993). P300/CBP is also involved in regulating inflammatory mediators (Deng et al., 2004, Blood WO 2016/044770 PCT/US2015/051028 103:2135-42; Tumer-Brannen et al., 2011, J. Immunol. 186:7127-7135). P300/CBP has also been linked to other diseases, such as fibrosis (Ghosh and Varga, 2007, J. Cell. Physiol. 213:663-671), metabolic syndrome (Bricambert et al., 2010, J. Clin. Invest. 120:4316-4331), and progressive neurodegenerative diseases, such as Huntington Disease (Cong et al., 2005, Mol. Cell. Neurosci. 30:12-23), Kennedy's disease (Lieberman et al., 2002, Hum. Mol. Genet. 11:1967-76), and Alzheimer's disease (Francis et al., 2007, Neurosci. Lett. 413:137-140).

The association of p300/CBP activity in disease pathogenesis suggests potential utility of p300/CBP as a therapeutic target. However, the identification of potent, specific histone acetyltransferase inhibitors has been challenging (Cole, 2008, Nat. Chem. Biol. 4:590-97). P300 HAT inhibitors derived from natural compounds have moderate potency but lack specificity (Dekker and Haisma, 2009, Dmg Disc. Today 14:942-8). Lys-CoA, converted to a cell-permeable form with a Tat peptide attachment, is more selective, but has limited use in pharmacological studies due to its complexity. Recently, a selective p300 inhibitor C646 was identified using the Lys-CoA/p300 HAT structure in a virtual ligand screening approach (Bowers et al., 2010, Chemistry & Biology 17:471-482). While progress has been made in this field, there remains a need in the art for improved HAT inhibitors.

WO 2016/196117 discloses N-pyridinyl-acetamide derivatives useful for the treatment of cancer.

### SUMMARY

Provided herein are compounds having the Formula **I:** 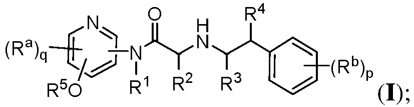 and pharmaceutically acceptable salts and compositions thereof, wherein R¹, R², R³, R⁴, R⁵, R^{a}, R^{b}, q, and p are as described herein. The disclosed compounds and compositions modulate histone acetyltranferases (see e.g., **Table 2**), and are useful in a variety of therapeutic applications such as, for example, in treating cancer. The invention is defined in the appended claims.

### DETAILED DESCRIPTION

### 1. General Description of Compounds

Provided herein is a compound of Formula **I**: 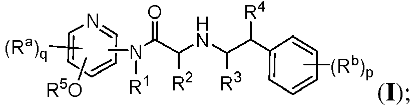 or a pharmaceutically acceptable salt thereof, wherein
R¹, R³, and R⁴ are each independently hydrogen or C₁₋₄alkyl;
R² is phenyl or 5- to 6-membered heteroaryl, each of which are optionally substituted
with 1 to 3 groups selected from R^{c};
R⁵ is a C₁₋₆alkyl substituted with 4- to 6-membered heterocyclyl or 5- to 6-membered heteroaryl, wherein each of said 4- to 6-membered heterocyclyl or 5- to 6-membered heteroaryl are optionally substituted with 1 to 3 groups selected from R^{d}; or R⁵ is 4- to 6-membered heterocyclyl or 4- to 6-membered heteroaryl, wherein each of said 4- to 6-membered heterocyclyl or 5- to 6-membered heteroaryl are optionally substituted with 1 to 3 groups selected from R^{d};
R^{a}, R^{b}, R^{c}, and R^{d} are each independently halo, CN, oxo, NO₂, C₁₋₆alkyl, C₂₋₆alkenyl, C₁₋₆alkoxy, halo(C₁₋₆alkoxyl), halo(C₁₋₆alkyl), -C₁₋₆alkylOR^{e}, -C(O)R^{f}, -C(O)OR, -C₁₋₆alkylC(O)OR^{e}, -C(O)N(R^{e})₂, -C(O)NR^{e}C₁₋₆alkylOR^{e}, -OC₁₋₆alkylN(R^{e})₂, -C₁₋₆alkylC(O)N(R^{e})₂, -C₁₋₆alkylN(R^{e})₂, -N(R^{e})₂, -C(O)NR^{e}C₁₋₆alkylN(R^{e})₂, -NR^{e}C₁₋₆alkylN(R^{e})₂. -NR^{e}C₁₋₆alkylOR^{e}, -SOR^{e}, -S(O)₂R^{e}, -SON(R^{e})₂, -SO₂N(R^{e})₂, SF₅, -O(C₃₋C₆)cycloalkyl, -O-C₁₋₄alkylaryl, -C₁₋₆alkyl(C₃₋C₆)cycloalkyl, -C₁₋₆alkylaryl, -C₁₋₆alkylheteroaryl, -C₁₋₆alkylheterocyclyl, (C₃₋C₆)cycloalkyl, heterocyclyl, heteroaryl, or aryl, wherein each of said (C₃₋C₆)cycloalkyl, heterocyclyl, aryl, and heteroaryl alone and in connection with -O(C₃₋C₆)cycloalkyl, -C₁₋₆alkyl(C₃₋C₆)cycloalkyl, -C₁₋₆alkylaryl, -C₁₋₆alkylheteroaryl, and -C₁₋₆alkylheterocyclyl are optionally substituted with 1 to 3 groups selected from halo, C₁₋₆alkyl, C₁₋₆haloalkyl, C₁₋₆alkoxy, C₁₋₆haloalkoxy, -N(R^{e})₂, -C(O)R^{f}, and -C₁₋₆alkylOR^{e};
each R^{e} is independently hydrogen, halo(C₁₋₄alkyl), or C₁₋₄alkyl;
each R^{f} is independently hydrogen, halo(C₁₋₄alkyl), C₁₋₄alkyl, or (C₃₋C₄)cycloalkyl;
q is 0, 1, or 2; and
p is 0, 1, 2, or 3.

According to the invention,
R¹, R³, and R⁴ are each independently hydrogen or C₁₋₄alkyl;
R² is phenyl or pyrazolyl, each of which are optionally substituted with halo or C₁₋₄alkyl;
R⁵ is a C₁₋₆alkyl substituted with 4- to 6-membered heterocyclyl or 5- to 6-membered heteroaryl, wherein each of said 4- to 6-membered heterocyclyl or 5- to 6-membered heteroaryl are optionally substituted with 1 to 3 groups selected from R^{d}; or R⁵ is 4- to 6-membered heterocyclyl or 5- to 6-membered heteroaryl, wherein each of said 4- to 6-membered heterocyclyl or 5- to 6-membered heteroaryl are optionally substituted with 1 to 3 groups selected from R^{d};
R^{a}, if present, is C₁₋₃alkyl, C₁₋₃alkoxy, halo(C₁₋₃alkyl), haloC₁₋₃alkoxy, or halo;
R^{b} is halo, cyano, or -SO2NH₂;
each R^{d} is independently halo, CN, oxo, NO2, C₁₋₆alkyl, C2-6alkenyl, C₁₋₆alkoxy, halo(C₁₋₆alkoxy), halo(C₁₋₆alkyl), -C₁₋₆alkylOR^{e}, -C(O)R^{f}, -C(O)OR, -C₁₋₆alkylC(O)OR^{e}, -C(O)N(R^{e})₂, -C(O)NR^{e}C₁₋₆alkylOR^{e}, -OC₁₋₆alkylN(R^{e})₂, -C₁₋₆alkylC(O)N(R^{e})2, -C₁₋₆alkylN(R^{e})2, -N(R^{e})2, -C(O)NR^{e}C₁₋₆alkylN(R^{e})2, -NR^{e}C₁₋₆alkylN(R^{e})₂,-NR^{e}C₁₋₆alkylOR^{e}, - SOR^{e}, -S(O)₂R^{e}, -SON(R^{e})₂, -SO₂N(R^{e})₂, -O(C₃-C₆)cycloalkyl, -O-C₁₋₄alkylaryl, -C₁₋₆ alkyl(C₃-C₆)cycloalkyl, -C₁₋₆alkylaryl, -C₁₋₆alkylheteroaryl, -C₁₋₆alkylheterocyclyl, (C₃₋C₆)cycloalkyl, heterocyclyl, heteroaryl, or aryl, wherein each of said (C₃₋C₆)cycloalkyl, heterocyclyl, aryl, and heteroaryl alone and in connection with -O(C₃-C₆)cycloalkyl, - C₁₋₆alkyl(C₃-C₆)cycloalkyl, -C₁₋₆alkylaryl, -C₁₋₆alkylheteroaryl, and-C₁₋₆alkylheterocyclyl are optionally substituted with 1 to 3 groups selected from halo, C₁₋₆alkyl, C₁₋₆haloalkyl, C1-6alkoxy, C1-6haloalkoxy, -N(R^{e})₂, -C(O)R^{f}, and -C₁₋₆alkylOR^{e};
each R^{e} is independently hydrogen, halo(C₁₋₄alkyl), or C₁₋₄alkyl;
each R^{f} is independently hydrogen, halo(C₁₋₄alkyl), C₁₋₄alkyl, or (C₃-C₄)cycloalkyl;
q is 0 or 1;
and p is 1.

### 2. Definitions

When used in connection to describe a chemical group that may have multiple points of attachment, a hyphen (-) designates the point of attachment of that group to the variable to which it is defined. For example, -N(R^{d})₂ and -NR^{d}C₁₋₆alkylOR^{d} mean that the point of attachment for this group occurs on the nitrogen atom.

The terms "halo" and "halogen" refer to an atom selected from fluorine (fluoro, -F), chlorine (chloro, -Cl), bromine (bromo, -Br), and iodine (iodo, -I).

The term "alkyl" when used alone or as part of a larger moiety, such as "haloalkyl", means saturated straight-chain or branched monovalent hydrocarbon radical. Unless otherwise specified, an alkyl group typically has 1-6 carbon atoms, *i.e*.*,* (C₁-C₆)alkyl.

"Alkoxy" means an alkyl radical attached through an oxygen linking atom, represented by -O-alkyl. For example, "(C₁-C₄)alkoxy" includes methoxy, ethoxy, proproxy, and butoxy.

The term "haloalkyl" includes mono, poly, and perhaloalkyl groups where the halogens are independently selected from fluorine, chlorine, bromine, and iodine.

"Haloalkoxy" is a haloalkyl group which is attached to another moiety via an oxygen atom such as, e.g., but are not limited to -OCHCF₂ or -OCF₃.

The term "oxo" refers to =O.

The term "aryl" refers to an aromatic carbocyclic single ring or two fused ring system containing 6 to 10 carbon atoms. Examples include phenyl, indanyl, tetrahydronaphthalene, and naphthyl.

The term "cycloalkyl" refers to a 4- to 12-membered monocyclic, bicyclic (e.g., a bridged or spiro bicyclic ring), polycyclic (e.g., tricyclic), or fused hydrocarbon ring system that is completely saturated. Monocyclic cycloalkyl groups include, without limitation, cyclopropyl, cyclobutyl, cyclopentyl, cyclohexyl, cycloheptyl, and cyclooctyl. Bridged bicyclic cycloalkyl groups include, without limitation, bicyclo[3.2.1]octane, bicyclo[2.2.1]heptane, bicyclo[3.1.0]hexane, bicyclo[1.1.1]pentane, and the like. Spiro bicyclic cycloalkyl groups include, e.g., spiro[3.6]decane, spiro[4.5]decane, and the like. Fused cycloalkyl rings include, e.g., decahydronaphthalene, octahydropentalene, and the like. It will be understood that when specified, optional substituents on a cycloalkyl may be present on any substitutable position and, include, *e.g.,* the position at which the cycloalkyl group is attached. In one aspect, a cycloalkyl is a C₃-C₆ monocyclic hydrocarbon ring system that is completely saturated.

The term "heteroaryl" used alone or as part of a larger moiety refers to a 5- to 12-membered (e.g., a 5- to 7-membered or 5- to 6-membered) aromatic radical containing 1-4 heteroatoms selected from N, O, and S. A heteroaryl group may be mono- or bi-cyclic. Monocyclic heteroaryl includes, for example, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, etc. Bi-cyclic heteroaryls include groups in which a monocyclic heteroaryl ring is fused to one or more aryl or heteroaryl rings. Nonlimiting examples include indolyl, imidazopyridinyl, benzooxazolyl, benzooxodiazolyl, indazolyl, benzimidazolyl, benzthiazolyl, quinolyl, quinazolinyl, quinoxalinyl, pyrrolopyridinyl, pyrrolopyrimidinyl, pyrazolopyridinyl, thienopyridinyl, thienopyrimidinyl, indolizinyl, purinyl, naphthyridinyl, and pteridinyl. It will be understood that when specified, optional substituents on a heteroaryl group may be present on any substitutable position and, include, *e.g.,* the position at which the heteroaryl is attached.

The term "heterocyclyl" means a 4- to 12-membered (e.g., a 4- to 7-membered or 4- to 6-membered) saturated or partially unsaturated heterocyclic ring containing 1 to 4 heteroatoms independently selected from N, O, and S. It can be mononcyclic, bicyclic (e.g., a bridged, fused, or spiro bicyclic ring), or tricyclic. A heterocyclyl ring can be attached to its pendant group at any heteroatom or carbon atom that results in a stable structure. Examples of such saturated or partially unsaturated heterocyclic radicals include, without limitation, tetrahydrofuranyl, tetrahydrothienyl, terahydropyranyl, pyrrolidinyl, pyridinonyl, pyrrolidonyl, piperidinyl, oxazolidinyl, piperazinyl, dioxanyl, dioxolanyl, morpholinyl, dihydrofuranyl, dihydropyranyl, dihydropyridinyl, tetrahydropyridinyl, dihydropyrimidinyl, oxetanyl, azetidinyl and tetrahydropyrimidinyl. A heterocyclyl group may be mono- or bicyclic. The term "heterocyclyl" also includes, e.g., unsaturated heterocyclic radicals fused to another unsaturated heterocyclic radical or aryl or heteroaryl ring, such as for example, tetrahydronaphthyridine, indolinone, dihydropyrrolotriazole, imidazopyrimidine, quinolinone, dioxaspirodecane. It will also be understood that when specified, optional substituents on a heterocyclyl group may be present on any substitutable position and, include, *e.g.,* the position at which the heterocyclyl is attached (e.g., in the case of an optionally substituted heterocyclyl or heterocyclyl which is optionally substituted).

The term "spiro" refers to two rings that shares one ring atom (e.g., carbon).

The term "fused" refers to two rings that share two adjacent ring atoms with one another.

The term "bridged" refers to two rings that share three ring atoms with one another.

The disclosed compounds exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that contain two or more asymmetrically substituted carbon atoms. "R" and "S" represent the configuration of substituents around one or more chiral carbon atoms.

A hash bond as in " 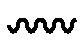 " represents the point at which the depicted group is attached to the defined variable. For example, if R⁵ is defined as 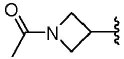 in a compound having the Formula **I**, that means the resulting compound is of the following formula:

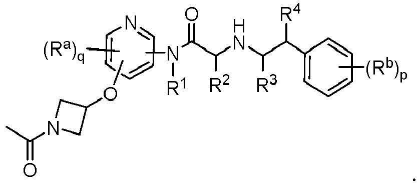

"Racemate" or "racemic mixture" means a compound of equimolar quantities of two enantiomers, wherein such mixtures exhibit no optical activity, *i.e*., they do not rotate the plane of polarized light.

The compounds of the herein may be prepared as individual enantiomers by either enantio-specific synthesis or resolved from an enantiomerically enriched mixture. Conventional resolution techniques include forming the salt of a free base of each isomer of an enantiomeric pair using an optically active acid (followed by fractional crystallization and regeneration of the free base), forming the salt of the acid form of each enantiomer of an enantiomeric pair using an optically active amine (followed by fractional crystallization and regeneration of the free acid), forming an ester or amide of each of the enantiomers of an enantiomeric pair using an optically pure acid, amine or alcohol (followed by chromatographic separation and removal of the chiral auxiliary), or resolving an enantiomeric mixture of either a starting material or a final product using various well known chromatographic methods. Additionally, the compounds can be prepared as individual enantiomers by separating a racemic mixture using conventional chiral chromatography techniques.

When the stereochemistry of a disclosed compound is named or depicted by structure, the named or depicted stereoisomer is at least 60%, 70%, 80%, 90%, 99% or 99.9% by weight pure relative to all of the other stereoisomers. Percent by weight pure relative to all of the other stereoisomers is the ratio of the weight of one stereoisomer over the weight of the other stereoisomers. When a single enantiomer is named or depicted by structure, the depicted or named enantiomer is at least 60%, 70%, 80%, 90%, 99% or 99.9% by weight optically pure. Percent optical purity by weight is the ratio of the weight of the enantiomer over the weight of the enantiomer plus the weight of its optical isomer.

When the stereochemistry of a disclosed compound is named or depicted by structure, and the named or depicted structure encompasses more than one stereoisomer (e.g., as in a diastereomeric pair), it is to be understood that one of the encompassed stereoisomers or any mixture of the encompassed stereoisomers are included. It is to be further understood that the stereoisomeric purity of the named or depicted stereoisomer is at least 60%, 70%, 80%, 90%, 99% or 99.9% by weight pure relative to all of the other stereoisomers. The stereoisomeric purity in this case is determined by dividing the total weight in the mixture of the stereoisomers encompassed by the name or structure by the total weight in the mixture of all of the stereoisomers. Unless otherwise specified, if only some of the stereochemical centers in a disclosed compound are depicted or named by structure, the named or depicted configuration is enriched relative to the remaining configurations, for example, by a molar excess of at least 60%, 70%, 80%, 90%, 99% or 99.9%. For example, the structure: 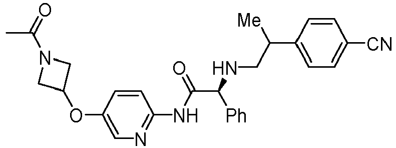 means that that the configuration about the chiral carbon where the stereochemistry is depicted is stereochemically enriched as S (e.g., by a molar excess of at least 60%, 70%, 80%, 90%, 99% or 99.9%) and that the stereochemistry at the other chiral center, to which the stereochemistry is not identified, may be R or S, or a mixture thereof.

When a disclosed compound is named or depicted by structure without indicating the stereochemistry, and the compound has one chiral center, it is to be understood that the name or structure encompasses one enantiomer of compound free from the corresponding optical isomer, a racemic mixture of the compound, or mixtures enriched in one enantiomer relative to its corresponding optical isomer.

When a disclosed compound is named or depicted by structure without indicating the stereochemistry and *e.g.,* the compound has more than one chiral center (e.g., at least two chiral centers), it is to be understood that the name or structure encompasses one stereoisomer free of other stereoisomers, mixtures of stereoisomers, or mixtures of stereoisomers in which one or more stereoisomers is enriched relative to the other stereoisomer(s). For example, the name or structure may encompass one stereoisomer free of other diastereomers, mixtures of stereoisomers, or mixtures of stereoisomers in which one or more diastereomers is enriched relative to the other diastereomer(s).

The terms "subject" and "patient" may be used interchangeably, and means a mammal in need of treatment, *e.g.,* companion animals (*e.g.*, dogs, cats, and the like), farm animals (*e.g.*, cows, pigs, horses, sheep, goats and the like) and laboratory animals (*e.g.*, rats, mice, guinea pigs and the like). Typically, the subject is a human in need of treatment.

The term "inhibit," "inhibition" or "inhibiting" includes a decrease in the baseline activity of a biological activity or process.

As used herein, the terms "treatment," "treat," and "treating" refer to reversing, alleviating, delaying the onset of, or inhibiting the progress of a disease or disorder, or one or more symptoms thereof, as described herein. In some aspects, treatment may be administered after one or more symptoms have developed, *i.e*., therapeutic treatment. In other aspects, treatment may be administered in the absence of symptoms. For example, treatment may be administered to a susceptible individual prior to the onset of symptoms (e.g., in light of a history of symptoms and/or in light of exposure to a particular organism, or other susceptibility factors), *i.e*., prophylactic treatment. Treatment may also be continued after symptoms have resolved, for example to delay their recurrence.

The term "pharmaceutically acceptable carrier" refers to a non-toxic carrier, adjuvant, or vehicle that does not destroy the pharmacological activity of the compound with which it is formulated. Pharmaceutically acceptable carriers, adjuvants or vehicles that may be used in the compositions described herein include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The term "effective amount" or "therapeutically effective amount" refers to an amount of a compound described herein that will elicit a biological or medical response of a subject e.g., a dosage of between 0.01 - 100 mg/kg body weight/day.

### 3. Compounds

In a first embodiment, provided herein is a compound of Formula **I:** 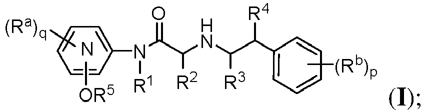 or a pharmaceutically acceptable salt thereof, wherein the variables are as described above or in the appended claims for the compounds of the invention.

In a second embodiment, the compound of Formula **I** is of the Formula **II:** 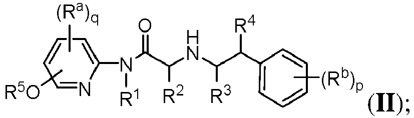 or a pharmaceutically acceptable salt thereof, wherein the remaining variables are as described for Formula I above, or in the appended claims for the compounds of the invention.

In a third embodiment, the compound of Formula **I** is of the Formula **III:** 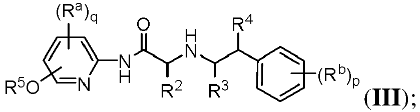 or a pharmaceutically acceptable salt thereof, wherein the remaining variables are as described for Formula I above, or in the appended claims for the compounds of the invention.

In a fourth embodiment, the compound of Formula **I** is of the Formula **IV:** 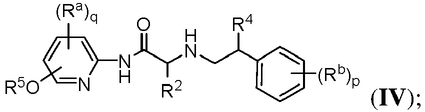 or a pharmaceutically acceptable salt thereof, wherein the remaining variables are as described for Formula I above, or in the appended claims for the compounds of the invention.

In a fifth embodiment, which is according to the invention, q in the compound of Formula **I**, **II**, **III**, or **IV** is 0 or 1.

In a sixth embodiment, which is according to the invention, R^{a}, if present, in the compound of Formula **I**, **II**, **III**, or **IV** is C₁₋₃alkyl, C₁₋₃alkoxy, halo(C₁₋₃alkyl), haloC₁₋₃alkoxy, or halo.

In a seventh embodiment, the compound of Formula **I** is of the Formula **V:** 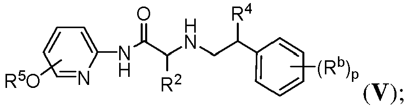 or a pharmaceutically acceptable salt thereof, wherein the remaining variables are as described for Formula I above, or in the appended claims for the compounds of the invention.

In an eighth embodiment, the compound of Formula **I** is of the Formula **VI** or **VII:** 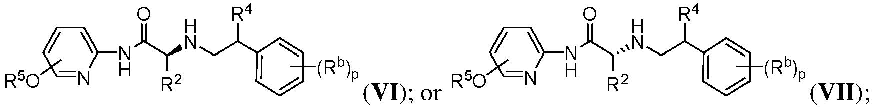 or a pharmaceutically acceptable salt thereof, wherein the remaining variables are as described for Formula I above, or in the appended claims for the compounds of the invention.

In a ninth embodiment, the compound of Formula **I** is of the Formula **VIII**, **IX**, **X**, or **XI:** 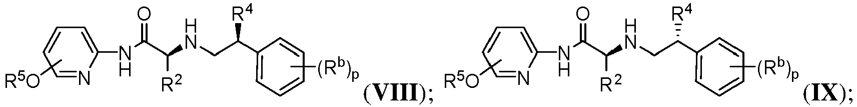 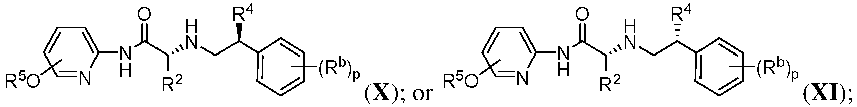 or a pharmaceutically acceptable salt thereof, wherein the remaining variables are as described for Formula I above, or in the appended claims for the compounds of the invention.

In a tenth embodiment, the compound of Formula **I** is of the Formula **XII**, **XIII**, **XIV**, or **XV:** 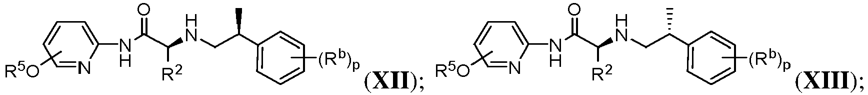 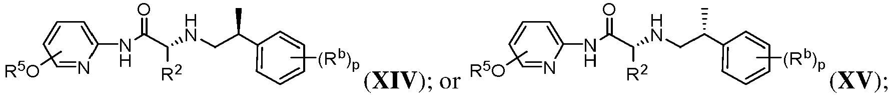 or a pharmaceutically acceptable salt thereof, wherein the remaining variables are as described for Formula I above, or in the appended claims for the compounds of the invention.

In an eleventh embodiment, the compound of Formula **I** is of the Formula **XVI** or **XVII:** 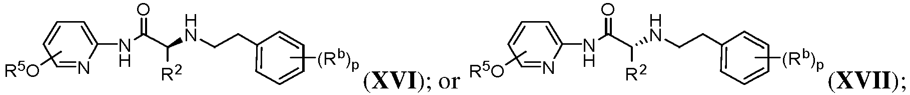 or a pharmaceutically acceptable salt thereof, wherein the remaining variables are as described for Formula I above, or in the appended claims for the compounds of the invention.

In a twelfth embodiment, R² in the compounds of Formula **I, II, III, IV, V, VI, VII, VIII, IX, X, XI, XII, XIII, XIV, XV, XVI,** or **XVII** is phenyl or pyrazolyl, each of which are optionally substituted with 1 to 3 groups selected from R^{c}, wherein the remaining variables are as described for Formula I above, or in the appended claims for the compounds of the invention.

In a thirteenth embodiment, R^{c} in the compounds of Formula **I, II, III, IV, V, VI, VII, VIII, IX, X, XI, XII, XIII, XIV, XV, XVI,** or **XVII** is halo, C₁₋₆alkyl, halo(C₁₋₆alkyl), C₁₋₆alkoxy, or halo(C₁₋₆alkoxy), wherein the remaining variables are as described for Formula I above, or in the appended claims for the compounds of the invention, or the twelfth embodiment.

In a fourteenth embodiment, which is according to the invention, R² in the compounds of Formula **I, II, III, IV, V, VI, VII, VIII, IX, X, XI, XII, XIII, XIV, XV, XVI,** or **XVII** is phenyl or pyrazolyl, each of which are optionally substituted with halo or C₁₋₄alkyl. Alternatively, R in the compounds of Formula **I, II, III, IV, V, VI, VII, VIII, IX, X, XI, XII, XIII, XIV, XV, XVI,** or **XVII** is phenyl.

In a fifteenth embodiment, which is according to the invention, p in the compounds of Formula **I, II, III, IV, V, VI**, **VII, VIII, IX, X, XI, XII, XIII, XIV, XV, XVI,** or **XVII** is 1.

In a sixteenth embodiment, which is according to the invention, R^{b} in the compounds of Formula **I, II, III, IV, V, VI, VII, VIII, IX, X, XI, XII, XIII, XIV, XV, XVI,** or **XVII** is halo, cyano, or -SO₂NH₂. Alternatively, R^{b} in the compounds of Formula **I, II, III, IV, V, VI, VII, VIII, IX, X, XI, XII, XIII, XIV, XV, XVI,** or **XVII** is cyano.

In a seventeenth embodiment, which is according to the invention, R⁵ in the compounds of Formula **I, II, III, IV, V, VI, VII, VIII, IX, X, XI, XII, XIII, XIV, XV, XVI,** or **XVII** is a C₁₋₄alkyl substituted with pyrazolyl or oxazolidinyl, each of which are optionally substituted with 1 to 3 groups selected from R^{d}; or R⁵ is piperidinyl, azetidinyl, hexahydropyrimidinyl, tetrahydrofuranyl, tetrahydropyranyl, oxetanyl, pyrazolyl, pyrrolidinyl, or pyrimidinyl, each of which are optionally substituted with 1 to 3 groups selected from R^{d}, wherein the remaining variables are as described for Formula I above, or in the appended claims for the compounds of the invention, or the twelfth, thirteenth, fourteenth, fifteenth, or sixteenth embodiment.

In an eighteenth embodiment, which is according to the invention, R^{d} in the compounds of Formula **I, II, III, IV, V, VI, VII, VIII, IX, X, XI, XII, XIII, XIV, XV, XVI,** or **XVII** is selected from halo, oxo, C₁₋₄alkyl, C₁₋₄alkoxy, halo(C₁₋₄alkyl), -C₁₋₄alkylOR^{e}, -C(O)R^{f}, -C(O)N(R^{e})₂, -C₁₋₆alkylC(O)N(R^{e})₂, and -S(O)₂R^{e}, wherein the remaining variables are as described for Formula I above, or in the appended claims for the compounds of the invention, or the twelfth, thirteenth, fourteenth, fifteenth, sixteenth, or seventeenth embodiment. Alternatively, R^{d} in the compounds of Formula **I, II, III, IV, V, VI, VII**, **VIII**, **IX, X, XI, XII, XIII, XIV, XV, XVI,** or **XVII** is selected from C₁₋₆alkyl, -C(O)R^{f}, and oxo, wherein the remaining variables are as described for Formula I above, or in the appended claims for the compounds of the invention, or the twelfth, thirteenth, fourteenth, fifteenth, sixteenth, or seventeenth embodiment.

In a ninteenth embodiment, which is according to the invention, R^{e} in the compounds of Formula **I, II, III, IV, V, VI, VII, VIII, IX, X, XI, XII, XIII, XIV, XV, XVI,** or **XVII** is hydrogen or C₁₋₃alkyl, wherein the remaining variables are as described for Formula I above, or in the appended claims for the compounds of the invention. or the twelfth, thirteenth, fourteenth, fifteenth, sixteenth, seventeenth, or eighteenth embodiment.

In a twentieth embodiment, which is according to the invention, R^{f} in the compounds of Formula **I, II, III, IV, V, VI**, **VII, VIII, IX, X, XI, XII, XIII, XIV, XV, XVI,** or **XVII** is cyclopropyl or C₁₋₄alkyl, wherein the remaining variables are as described for Formula I above, or in the appended claims for the compounds of the invention, or the twelfth , thirteenth, fourteenth, fifteenth, sixteenth, seventeenth, eighteenth, or nineteenth embodiment.

In a twenty-first embodiment, which is according to the invention, R⁵ in the compounds of Formula **I, II, III, IV, V, VI**, **VII**, **VIII**, **IX, X, XI, XII, XIII, XIV, XV, XVI,** or **XVII** is 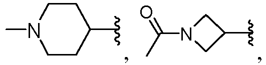 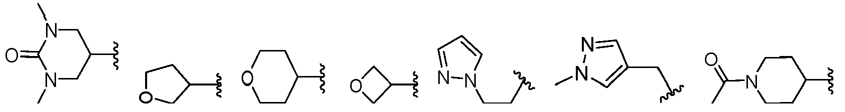 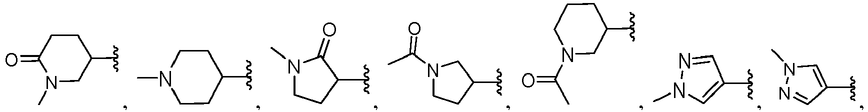 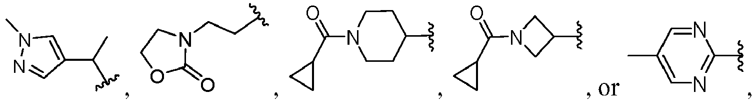 wherein the remaining variables are as described for Formula I above, or in the appended claims for the compounds of the invention, or the twelfth, thirteenth, fourteenth, fifteenth, sixteenth, seventeenth, eighteenth, nineteenth, or twentieth embodiment.

In a twenty-second embodiment, and according to the invention, the compound of Formula **I** is selected from the following formula: 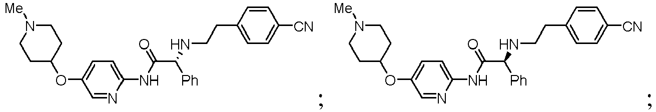 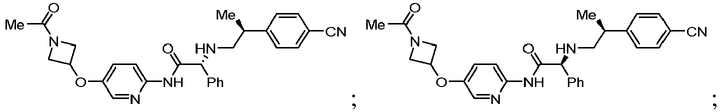 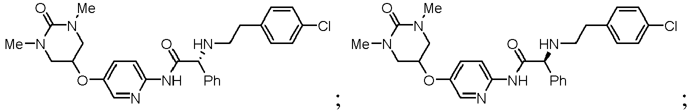 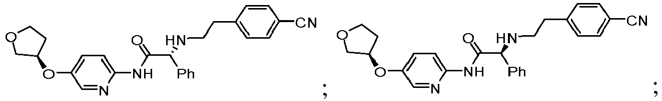 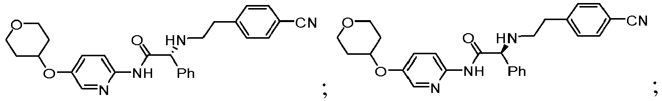 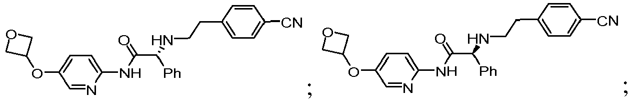 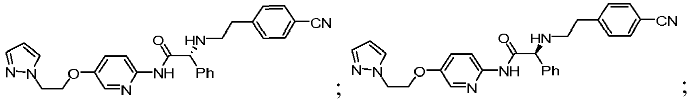 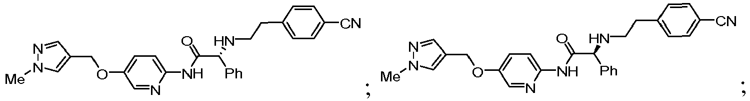 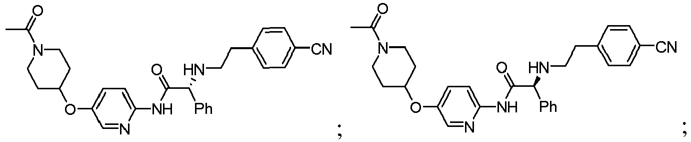 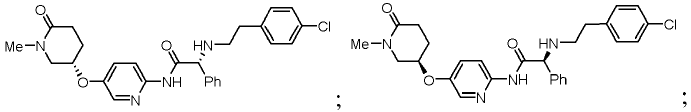 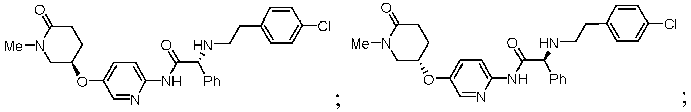 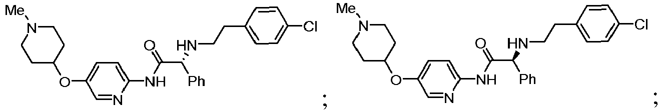 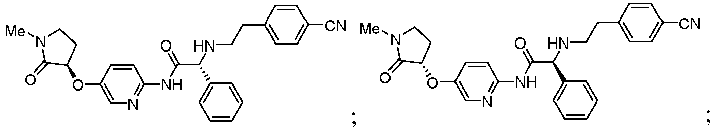 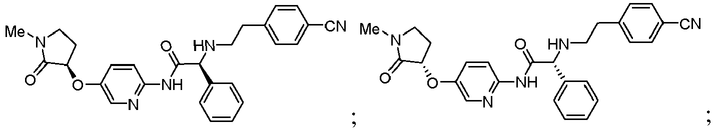 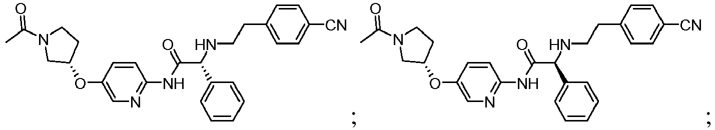 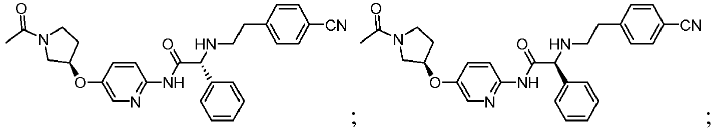 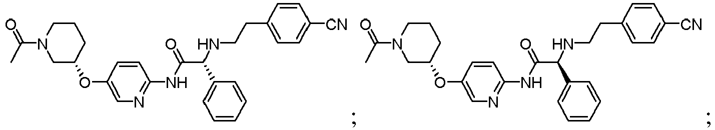 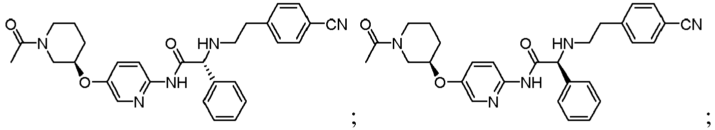 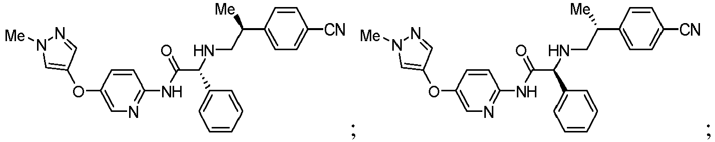 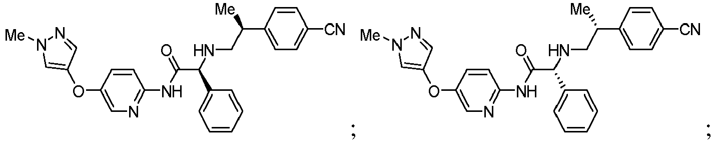 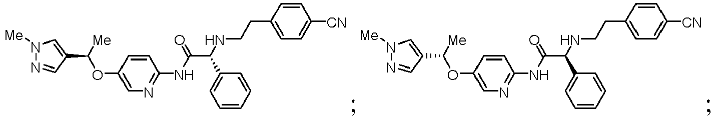 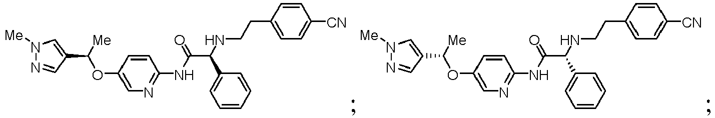 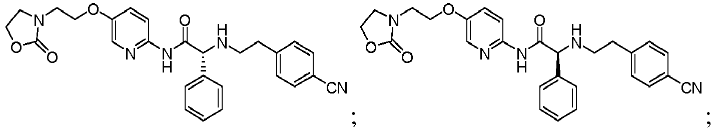 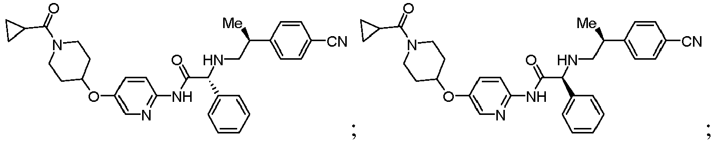 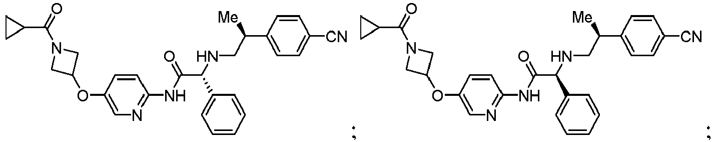 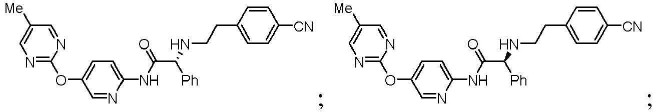 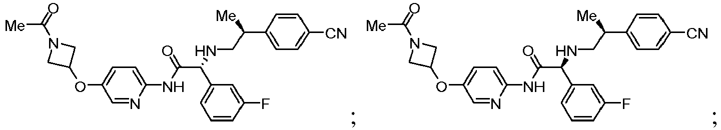 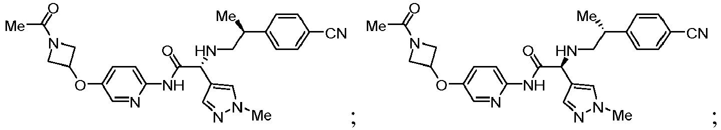 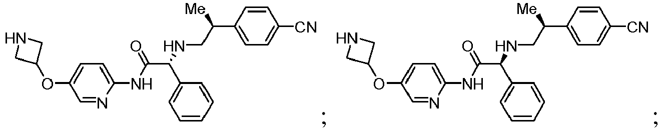 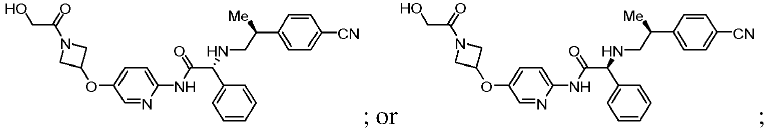 or a pharmaceutically acceptable salt thereof of any of the foregoing.

Specific examples of compounds are provided in the EXEMPLIFICATION section and are included as part of a twenty-third embodiment herein. Pharmaceutically acceptable salts as well as the neutral forms of these compounds are also included.

Also provided herein are pharmaceutical compositions comprising a compound described herin; and a pharmaceutically acceptable carrier.

### 4. Uses, Formulation and Administration

The references to methods of treatment in the subsequent paragraphs are to be interpreted as references to the compounds, pharmaceutical compositions and medicaments of the present invention for use in a method for treatment of the human or animal body by therapy or for diagnosis.

Compounds and compositions described herein are generally useful for modulating the activity of p300 and/or CBP HAT. In some aspects, the compounds and compositions described herein inhibit the activity of p300 and/or CBP HAT.

In some aspects, compounds and compositions described herein are useful in treating a disorder associated with p300 and/or CBP HAT function. Thus, provided herein are methods of treating a disorder associated with p300 and/or CBP HAT function, comprising administering to a subject in need thereof, a therapeutically effective amount of a compound described herein, or a pharmaceutically acceptable salt thereof, or a composition comprising a disclosed compound or pharmaceutically acceptable salt thereof. Also provided is the use of a compound described herein, or a pharmaceutically acceptable salt thereof, or a composition comprising a disclosed compound or pharmaceutically acceptable salt thereof, for the manufacture of a medicament for treating a disorder associated with p300 and/or CBP HAT function. Also provided is a compound described herein, or a pharmaceutically acceptable salt thereof, or a composition comprising a disclosed compound or pharmaceutically acceptable salt thereof, for use in treating a disorder associated with p300 and/or CBP HAT.

In some aspects, compounds and compositions described herein are useful in treating a disorder associated with chromatin acetylation at H3K27, H3K18, and other acetylation sites on the basic residues of chromatin acted upon by the p300 and/or CBP enzyme. Thus, provided herein are methods of treating a disorder associated with chromatin acetylation at H3K27, H3K18, and other acetylation sites on the basic residues of chromatin acted upon by the p300 and/or CBP enzyme, comprising administering to a subject in need thereof, a therapeutically effective amount of a compound described herein, or a pharmaceutically acceptable salt thereof, or a composition comprising a disclosed compound or pharmaceutically acceptable salt thereof. Also provided is the use of a compound described herein, or a pharmaceutically acceptable salt thereof, or a composition comprising a disclosed compound or pharmaceutically acceptable salt thereof, for the manufacture of a medicament for treating a disorder associated with chromatin acetylation at H3K27, H3K18, and other acetylation sites on the basic residues of chromatin acted upon by the p300 and/or CBP enzyme. Also provided is a compound described herein, or a pharmaceutically acceptable salt thereof, or a composition comprising a disclosed compound or pharmaceutically acceptable salt thereof, for use in treating a disorder associated with chromatin acetylation at H3K27, H3K18, and other acetylation sites on the basic residues of chromatin acted upon by the p300 and/or CBP enzyme.

In some aspects, compounds and compositions described herein are useful in treating a disorder associated with hyperacetylation of chromatin and/or hyperacetylation of proteins that are known to be acetylated by p300 and/or CBP. Thus, provided herein are methods of treating a disorder associated with hyperacetylation of chromatin and/or hyperacetylation of proteins that are known to be acetylated by p300 and/or CBP, comprising administering to a subject in need thereof, a therapeutically effective amount of a compound described herein, or a pharmaceutically acceptable salt thereof, or a composition comprising a disclosed compound or pharmaceutically acceptable salt thereof. Also provided is the use of a compound described herein, or a pharmaceutically acceptable salt thereof, or a composition comprising a disclosed compound or pharmaceutically acceptable salt thereof, for the manufacture of a medicament for treating a disorder associated with hyperacetylation of chromatin and/or hyperacetylation of proteins that are known to be acetylated by p300 and/or CBP. Also provided is a compound described herein, or a pharmaceutically acceptable salt thereof, or a composition comprising a disclosed compound or pharmaceutically acceptable salt thereof, for use in treating a disorder associated with hyperacetylation of chromatin and/or hyperacetylation of proteins that are known to be acetylated by p300 and/or CBP.

In some aspects, the compounds and compositions described herein are useful in treating cancer, cardiac disease, metabolic disease, fibrotic disease, inflammatory disease, or viral infections.

In some aspects, the cancer treated by the compounds and compositions described herein is selected from adenocarcinoma of the breast, prostate, and colon; bronchogenic carcinoma of the lung; myeloid; melanoma; hepatoma; neuroblastoma; papilloma; apudoma; choristoma; branchioma; malignant carcinoid syndrome; carcinoid heart disease; carcinoma (e.g., Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell); histiocytic disorders; leukemia; histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor; adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin; angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

In other aspects, the cancer treated by the compounds and compositions described herein is selected from acoustic neuroma, acute leukemia, acute lymphocytic leukemia, acute myelocytic leukemia, acute T-cell leukemia, basal cell carcinoma, bile duct carcinoma, bladder cancer, brain cancer, breast cancer, bronchogenic carcinoma, cervical cancer, chondrosarcoma, chordoma, choriocarcinoma, chronic leukemia, chronic lymphocytic leukemia, chronic myelocytic leukemia, chronic myelogenous leukemia, colon cancer, colorectal cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen-receptor positive breast cancer, essential thrombocythemia, Ewing's tumor, fibrosarcoma, follicular lymphoma, germ cell testicular cancer, glioma, glioblastoma, gliosarcoma, heavy chain disease, head and neck cancer, hemangioblastoma, hepatoma, hepatocellular cancer, hormone insensitive prostate cancer, leiomyosarcoma, leukemia, liposarcoma, lung cancer, lymphagioendotheliosarcoma, lymphangiosarcoma, lymphoblastic leukemia, lymphoma, lymphoid malignancies of T- cell or B-cell origin, medullary carcinoma, medulloblastoma, melanoma, meningioma, mesothelioma, multiple myeloma, myelogenous leukemia, myeloma, myxosarcoma, neuroblastoma, NUT midline carcinoma (NMC), non-small cell lung cancer, oligodendroglioma, oral cancer, osteogenic sarcoma, ovarian cancer, pancreatic cancer, papillary adenocarcinomas, papillary carcinoma, pinealoma, polycythemia vera, prostate cancer, rectal cancer, renal cell carcinoma, retinoblastoma, rhabdomyosarcoma, sarcoma, sebaceous gland carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom's macroglobulinemia, testicular tumors, uterine cancer, and Wilms' tumor.

In some aspects, the cancer treated by the compounds and compositions described herein is selected from colon cancer, gastric cancer, thyroid cancer, lung cancer, leukemia, pancreatic cancer, melanoma, multiple melanoma, brain cancer, CNS cancer, renal cancer, prostate cancer, ovarian cancer, leukemia, and breast cancer.

In some aspects, the cancer treated by the compounds and compositions described herein is selected from lung cancer, breast cancer, pancreatic cancer, colorectal cancer, and melanoma.

In some aspects, the cancer treated by the compounds and compositions described herein is selected from prostate cancer, enhancer drive cancers, multiple myeloma, and lymphoma (e.g., mantle cell lymphoma). See e.g., Santer et al 2011, Mol Cancer Ther. 10: 1644-1655; Lasko et al, 2017, Nature. Oct 5;550(7674):128-132; Tie F, et al. 2009 Development 136:3131-3141; Bergsagel PL, Kuehl WM 2001, Oncogene, 20(40):5611-22; Chesi and Bergsagel 2013, Int J Hematol. 97(3): 313-323; and Jares P et al 2007, Nat Rev Cancer. 7(10):750-762.

In one aspect, the cardiac disease treated by the compound and compositions described herein is selected from cardiac hypertrophy and heart failure.

In one aspect, the metabolic disease treated by the compound and compositions described herein is selected from obesity, hepatic steatosis, dyslipidemia, hypertension, coronary heart disease, hepatic inflammation, and diabetes mellitus type 2.

In one aspect, the fibrotic disease treated by the compound and compositions described herein is selected from radiation-induced pneumonitis, radiation fibrosis, acute respiratory distress syndrome, chronic obstructive pulmonary disease, idiopathic pulmonary fibrosis, interstitial lung disease, myocardial infarction, ischemic stroke, ischemic kidney disease, transplant rejection, Leishmaniasis, type I diabetes, rheumatoid arthritis, chronic hepatitis, cirrhosis, inflammatory bowel disease, Crohn's disease, scleroderma, keloid, postoperative fibrosis, chemotherapy induced fibrosis (e.g., chemotherapy induced pulmonary fibrosis or ovarian cortical fibrosis), nephrogenic systemic fibrosis, retroperitoneal fibrosis, myelofibrosis, mediastinal fibrosis, cystic fibrosis, asbestosis, asthma, and pulmonary hypertension.

In one aspect, the inflammatory disease treated by the compound and compositions described herein is selected from asthma, inflammatory bowel disease (Crohn's disease or ulcerative colitis), chronic obstructive pulmonary disease, rheumatoid arthritis, and psoriasis. In another aspect, the inflammatory disease treated by the compound and compositions described herein is selected from Addison's disease, acute gout, ankylosing spondylitis, asthma, atherosclerosis, Behcet's disease, bullous skin diseases, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, Polyarteritis nodosa, pneumonitis, primary biliary cirrhosis, psoriasis, psoriatic arthritis, rheumatoid arthritis, scleritis, sclerosing cholangitis, sepsis, systemic lupus erythematosus, Takayasu's Arteritis, toxic shock, thyroiditis, type I diabetes, ulcerative colitis, uveitis, vitiligo, vasculitis, and Wegener's granulomatosis.

In one aspect, the viral infection treated by the compound and compositions described herein is selected from human immunodeficiency virus, hepatitis C virus, and human papilloma virus.

In one aspect, the compounds and compositions described herein are useful in treating neurological disorders.

Examples of neurological disorders include: (i) chronic neurodegenerative diseases such as fronto-temporal lobar degeneration (frontotemporal dementia, FTD), FTD-GRN, familial and sporadic amyotrophic lateral sclerosis (FALS and ALS, respectively), familial and sporadic Parkinson's disease, Parkinson's disease dementia, Huntington's disease, familial and sporadic Alzheimer's disease, multiple sclerosis, muscular dystrophy, olivopontocerebellar atrophy, multiple system atrophy, Wilson's disease, progressive supranuclear palsy, diffuse Lewy body disease, corticodentatonigral degeneration, progressive familial myoclonic epilepsy, striatonigral degeneration, torsion dystonia, familial tremor, Down's Syndrome, Gilles de la Tourette syndrome, Hallervorden-Spatz disease, peripheral neuropathy, diabetic peripheral neuropathy, dementia pugilistica, AIDS Dementia, age related dementia, age associated memory impairment, and amyloidosis-related neurodegenerative diseases such as those caused by the prion protein (PrP) which is associated with transmissible spongiform encephalopathy (Creutzfeldt-Jakob disease, Gerstmann-Straussler-Scheinker syndrome, scrapie, and kuru), and those caused by excess cystatin C accumulation (hereditary cystatin C angiopathy); and (ii) acute neurodegenerative disorders such as traumatic brain injury (e.g., surgery-related brain injury), cerebral edema, peripheral nerve damage, spinal cord injury, Leigh's disease, Guillain-Barre syndrome, lysosomal storage disorders such as lipofuscinosis, Alper's disease; pathologies arising with chronic alcohol or drug abuse including, for example, the degeneration of neurons in locus coeruleus and cerebellum, drug-induced movement disorders; pathologies arising with aging including degeneration of cerebellar neurons and cortical neurons leading to cognitive and motor impairments; and pathologies arising with chronic amphetamine abuse to including degeneration of basal ganglia neurons leading to motor impairments; pathological changes resulting from focal trauma such as stroke, focal ischemia, vascular insufficiency, hypoxic-ischemic encephalopathy, hyperglycemia, hypoglycemia or direct trauma; pathologies arising as a negative side-effect of therapeutic drugs and treatments (e.g., degeneration of cingulate and entorhinal cortex neurons in response to anticonvulsant doses of antagonists of the NMDA class of glutamate receptor) and Wernicke-Korsakoff's related dementia.

Other neurological disorders include nerve injury or trauma associated with spinal cord injury. Neurological disorders of limbic and cortical systems include e.g., cerebral amyloidosis, Pick's atrophy, and Rett syndrome. In another aspect, neurological disorders include disorders of mood, such as affective disorders and anxiety; disorders of social behavior, such as character defects and personality disorders; disorders of learning, memory, and intelligence, such as mental retardation and dementia. Thus, in one aspect the disclosed compounds and compositions may be useful in treating schizophrenia, delirium, attention deficit hyperactivity disorder (ADHD), schizoaffective disorder, Alzheimer's disease, vascular dementia, Rubinstein-Taybi syndrome, depression, mania, attention deficit disorders, drug addiction, dementia, and dementia including BPSD manifestations.

Further neurological conditions include e.g., tauopathies, spinal and bulbar muscular atrophy, spinocerebellar ataxia type 3, pain (including e.g., acute and chronic pain, somatic pain, visceral pain, neuropathic pain, peripheral neuropathy, nociceptive pain, central pain syndrome, muscular or joint pain), and neuroinflammation.

In one aspect, the compounds and compositions described herein are useful in treating a neurological disorder selected from frontotemporal dementia, Alzheimer's disease, tauopathies, vascular dementia, Parkinson's disease, and dementia with Lewy bodies.

In certain aspects, a composition described herein is formulated for administration to a patient in need of such composition. Compositions described herein may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. In some embodiments, the compositions are administered orally, intraperitoneally or intravenously. Sterile injectable forms of the compositions described herein may be aqueous or oleaginous suspension. These suspensions may be formulated according to techniques known in the art using suitable dispersing or wetting agents and suspending agents.

In some aspects, the compositions are administered orally.

A specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound described herein in the composition will also depend upon the particular compound in the composition.

The compounds described herein may be present in the form of pharmaceutically acceptable salts. For use in medicines, the salts of the compounds described herein refer to non-toxic "pharmaceutically acceptable salts." Pharmaceutically acceptable salt forms include pharmaceutically acceptable acidic/anionic or basic/cationic salts. Suitable pharmaceutically acceptable acid addition salts of the compounds described herein include e.g., salts of inorganic acids (such as hydrochloric acid, hydrobromic, phosphoric, nitric, and sulfuric acids) and of organic acids (such as, acetic acid, benzenesulfonic, benzoic, methanesulfonic, and p-toluenesulfonic acids). Compounds of the present teachings with acidic groups such as carboxylic acids can form pharmaceutically acceptable salts with pharmaceutically acceptable base(s). Suitable pharmaceutically acceptable basic salts include e.g., ammonium salts, alkali metal salts (such as sodium and potassium salts) and alkaline earth metal salts (such as magnesium and calcium salts). Compounds with a quaternary ammonium group also contain a counteranion such as chloride, bromide, iodide, acetate, perchlorate and the like. Other examples of such salts include hydrochlorides, hydrobromides, sulfates, methanesulfonates, nitrates, benzoates and salts with amino acids such as glutamic acid.

Combination therapies using a therapeutically effective amount of a compound of Formula **I**, or a pharmaceutically acceptable salt thereof, and an effective amount of one or more additional pharmaceutically active agents are also included herein. Additional active agents that can be combined with a compound of Formula **I**, or a pharmaceutically acceptable salt thereof, include e.g., those which target the estrogen receptor (ER). These include, but are not limited to selective estrogen receptor degraders (SERDs), ER antagonists, selective estrogen receptor modulators (SERMs), and aromatase inhibitors (AIs). Examples of SERDs and ER antagonists include, but are not limited to, fulvestrant, RAD-1901 (elacestrant), GDC-0927 ((2S)-2-(4-{2-[3-(fluoromethyl)-1-azetidinyl]ethoxy}phenyl)-3-(3-hydroxyphenyl)-4-methyl-2H-chromen-6-ol), GDC-0810 (brilanestrant), AZD-9496 ((2E)-3-[3,5-difluoro-4-[(1R,3R)-2-(2-fluoro-2-methylpropyl)-2,3,4,9-tetrahydro-3-methyl-1H-pyrido[3,4-b]indol-1-yl]phenyl]-2-propenoic acid), OP-1250 (a prodrug of (S)-3-(4-hydroxyphenyl)-4-methyl-2-(4-(2-((R)-3-methylpyrrolidin-1-yl)ethoxy)phenyl)-2H-chromen-7-ol found in US 9,018,244, the contents of which are incorporated herein by reference), (S)-3-(4-hydroxyphenyl)-4-methyl-2-(4-(2-((R)-3-methylpyrrolidin-1-yl)ethoxy)phenyl)-2H-chromen-7-ol, also found in US 9,018,244, the contents of which are incorporated herein by reference), LSZ102 ((E)-3-(4-((2-(2-(1,1-difluoroethyl)-4-fluorophenyl)-6-hydroxybenzo[b]thiophen-3-yl)oxy)phenyl)acrylic acid), and H3B-6545 ((E)-N,N-dimethyl-4-((2-((5-((Z)-4,4,4-trifluoro-1-(3-fluoro-1H-indazol-5-yl)-2-phenylbut-1-en-1-yl)pyridin-2-yl)oxy)ethyl)amino)but-2-enamide). Examples of SERMs include, but are not limited to, tamoxifen, toremifene, raloxifene, bazedoxifene, ospemifene, and nafoxidene. Examples of AIs include, but are not limited to, anastrozole, letrozole, exemestane, vorozole, formestane and fadrozole. In one aspect, provided is a compound of Formula **I**, or a pharmaceutically acceptable salt thereof, and an additional therapeutic agent selected from fulvestrant, RAD-1901, GDC-0927, GDC-0810, AZD-9496, OP-1250, LSZ102, H3B-6545, tamoxifen, toremifene, raloxifene, bazedoxifene, ospemifene, nafoxidene, anastrozole, letrozole, exemestane, vorozole, formestane and fadrozole. In one aspect, the additional therapeutic agent is fulvestrant. The use of one or more of the combination therapies discussed above for treating a condition recited herein is also included within the scope of the present disclosure. For example, in one aspect, the combination treatments meantion above are useful in the treatment of cancer e.g., breast cancer.

### EXEMPLIFICATION

Representative examples of the disclosed compounds are illustrated in the following non-limiting methods, schemes, and examples.

The following abbreviations have the indicated meanings: Ac = acetyl; ACN = acetonitrile; AcO acetate; BOC = *t*-butyloxycarbonyl; CBZ = carbobenzoxy; CDI = carbonyldiimidazole; DBU = 1,8-Diazabicycloundec-7-ene; DCC = 1,3-dicyclohexylcarbodiimide; DCE = 1,2-dichloroethane; DI = de-ionized; DIAD = Diisopropyl azodicarboxylate; DIBAL = diisobutyl aluminum hydride; DIPA = diisopropylamine; DIPEA or DIEA = N,N-diisoproylethylamine, also known as Hunig's base; DMA = dimethylacetamide; DMAP = 4-(dimethylamino)pyridine; DMF = dimethylformamide; DMP = Dess-Martin periodinane; DPPA = Diphenylphosphoryl azide; DPPP = 1,3-bis(diphenylphosphino)propane; Dtbbpy = 4,4 '-di-/e/7-butyl-2,2 ' -dipyridyl; EDC or EDCI = 1-(3-dimethylaminopropyl)-3-ethylcarbodiimide hydrochloride; EDTA = ethylenediaminetetraacetic acid, tetrasodium salt; EtOAc = ethyl acetate; FAB = fast atom bombardment; FMOC = 9-fluorenylmethoxycarbonyl; HFIP = hexafluoroisopropanol; HMPA = hexamethylphosphoramide; HATU=(9-(7-Azabenzotriazol-l-yl)-N, N, N, N-tetramethyluroniumhexafluorophosphate; HOAt = 1-Hydroxy-7-azabenzotriazole or 3H-[1,2,3]triazolo[4,5-b]pyridin-3-ol; HOBt = 1-hydroxybenzotriazole; HRMS = high resolution mass spectrometry; KHMDS = potassium hexamethyldisilazane; LC-MS = Liquid chromatography-mass spectrometry; LDA = lithium diisopropylamide; LiHMDS = lithium hexamethyldisilazane; MCPBA = meta-chloroperbenzoic acid; MMPP = magnesium monoperoxyphthlate hexahydrate; Ms = methanesulfonyl = mesyl; MsO = methanefulfonate = mesylate; MTBE = Methyl t-butyl ether; NBS = N-bromosuccinimide; NMM = 4-methylmorpholine; NMP = N-methylpyrroIidinone; NMR = Nuclear magnetic resonance; PCC = pyridinium chlorochromate; PDC = pyridinium dichromate; Ph = phenyl; PPTS = pyridinium p-toluene sulfonate; pTSA = p-toluene sulfonic acid; r.t./RT = room temperature; rac. = racemic; T3P = 2,4,6-Tripropyl-1,3,5,2,4,6-trioxatriphosphinane 2,4,6-trioxide; TEA = triethylamine; TFA = trifluoroacetic acid; TfO = trifluoromethanesulfonate = triflate; THF = tetrahydrofuran; TLC = thin layer chromatography; TMSC1 = trimethylsilyl chloride.

The progress of reactions was often monitored by TLC or LC-MS. The LC-MS was recorded using one of the following methods.

### METHOD-C3:

| | | | | |
|---|---|---|---|---|
| Mobile Phase | (A ) | 2 mM Ammonium acetate + 0.1% Formic Acid in Water | | |
| | (B) | 0.1% Formic Acid in Acetonitrile | | |
| Column | : | BEH C18 (50*2.1mm) 1.7 um | | |
| Column Flow | : | 0.55 ml/min | | |
| Gradient | : | Time (min) | % A | % B |
| | | 0.01 | 98 | 2 |
| | | 0.30 | 98 | 2 |
| | | 0.60 | 50 | 50 |
| | | 1.10 | 25 | 75 |
| | | 2.00 | 0 | 100 |
| | | 2.70 | 0 | 100 |
| | | 2.71 | 98 | 2 |
| | | 3.00 | 98 | 2 |

### PDS Method-J:

| | | | | |
|---|---|---|---|---|
| Mobile Phase | (A) | 5mM Ammonium Acetate + 0.1% Formic Acid in Water | | |
| | (B) | 0.1% Formic Acid in Acetonitrile | | |
| Column | : | BEH C18 (50*2.1mm), 1.7um or Equivalent | | |
| Column Flow | : | 0.45 ml/min | | |
| Gradient | : | Time (min) | % A | % B |
| | | 0.01 | 98 | 2 |
| | | 0.50 | 98 | 2 |
| | | 5.00 | 10 | 90 |
| | | 6.00 | 5 | 95 |
| | | 7.00 | 5 | 95 |
| | | 7.01 | 98 | 2 |
| | | 8.00 | 98 | 2 |

### Method-H:

| | | | | |
|---|---|---|---|---|
| Mobile Phase | (A) | 5mM Ammonium bicarbonate in water | | |
| | (B) | Acetonitrile | | |
| Column | : | X-Bridge C18 (50*4.6 mm), 3.5 um | | |
| Column Flow | : | 1.0 ml/min | | |
| Gradient | : | Time (min) | % A | % B |
| | | 0.01 | 95 | 5 |
| | | 5.00 | 10 | 90 |
| | | 5.80 | 5 | 95 |
| | | 7.20 | 5 | 95 |
| | | 7.21 | 95 | 5 |
| | | 10.00 | 95 | 5 |

### Method-F:

| | | | | |
|---|---|---|---|---|
| Mobile Phase | (A) | 10mM Ammonium Acetate in WATER | | |
| | (B) | 100% Acetonitrile | | |
| Column | : | X-Bridge C18 (150*4.6 mm), 5 um or Equivalent | | |
| Column Flow | : | 1.0 ml/min | | |
| Gradient | : | Time (min) | % A | % B |
| | | 0.01 | 90 | 10 |
| | | 5.00 | 10 | 90 |
| | | 7.00 | 0 | 100 |
| | | 11.00 | 0 | 100 |
| | | 11.01 | 90 | 10 |
| | | 12.00 | 90 | 10 |

### Method-G:

| | | | | |
|---|---|---|---|---|
| Mobile Phase | (A) | 10mM Ammonium Acetate in Water | | |
| | (B) | 100% Acetonitrile | | |
| Column | : | X-Bridge C18 (150*4.6 mm), 5 um or Equivalent | | |
| Column Flow | : | 1.0 ml/min | | |
| Gradient | : | Time (min) | % A | % B |
| | | 0.01 | 100 | 0 |
| | | 7.00 | 50 | 50 |
| | | 9.00 | 0 | 100 |
| | | 11.00 | 0 | 100 |
| | | 11.01 | 100 | 0 |
| | | 12.00 | 100 | 0 |

NMR was recorded at room temperature unless noted otherwise on Varian Inova 400 or 500 MHz spectrometers with the solvent peak used as the reference or on Bruker 300 or 400 MHz spectrometers with the TMS peak used as internal reference.

Unless otherwise stated, the absolute configuration of each eluting stereoisomer in the following examples was not identified.

The compounds described herein may be prepared using the following methods and schemes. Unless specified otherwise, all starting materials used are commercially available.

### Method 1

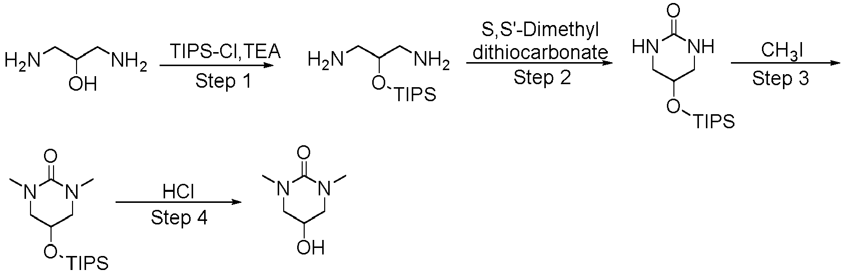

Method 1 is a four-step protocol for synthesis of 5-hydroxy-1,3-dimethyl tetrahydropyrimidin-2(1*H*)-one from 1,3-diaminopropan-2-ol that is useful for the synthesis of intermediates en route to the compounds described herein.

### Method 2

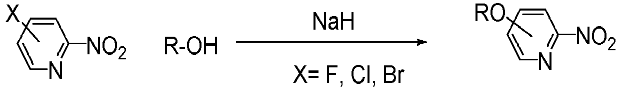

Method 2 is a protocol for the preparation of alkoxy-nitro pyridines, from the reaction of halonitropyridines with alcohols, that is useful for the synthesis of intermediates en route to the compounds described herein.

### Method 3

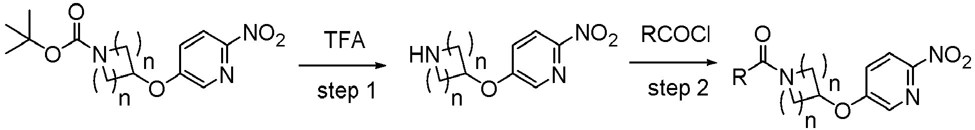

Method 3 is a two step protocol for the preparation of 1-(3-((6-nitropyridin-3-yl)oxy)heterocycl-1-yl)ethan-1-one derivatives that is useful for the synthesis of intermediates en route to the compounds described herein.

### Method 4

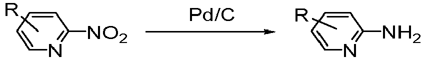

Method 4 is a protocol for the preparation of substituted 2-amino pyridines from 2-nitro pyridines via a palladium-catalyzed hydrogenation reaction that is useful for the synthesis of intermediates en route to the compounds described herein.

### Method 5

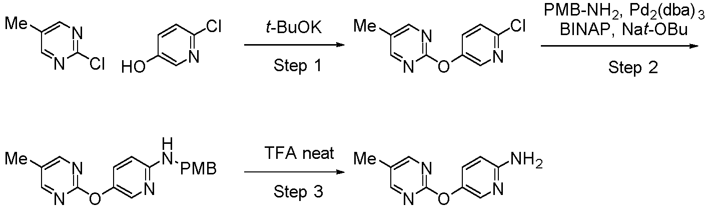

Method 5 is a three-step protocol for the preparation of pyrimidinyloxypyridine-2-amines from chloropyrimidines that is useful for the synthesis of intermediates en route to the compounds described herein.

### Method 6

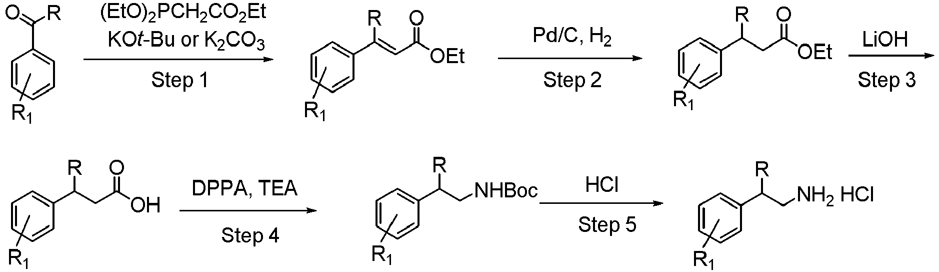

Method 6 is a five step-protocol for the preparation of substituted 2-arylethylamines and 2-heteroarylethylamines employing substituted benzaldehydes or ketones that is useful for the synthesis of intermediates en route to the compounds described herein.

### Method 7

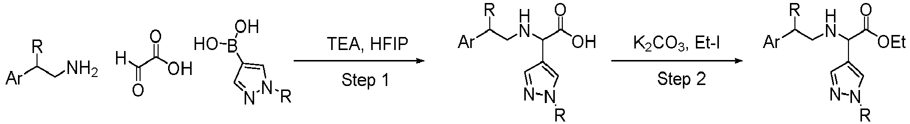

Method 7 is a two-step protocol, used for the preparation of substituted ethyl 2-(arylethylamino)-2-(1-substituted-1H-pyrazol-4-yl)acetates starting from arylethylamines and substituted boronate (or boronic acid)pyrazoles that is useful for the synthesis of intermediates en route to the compounds described herein.

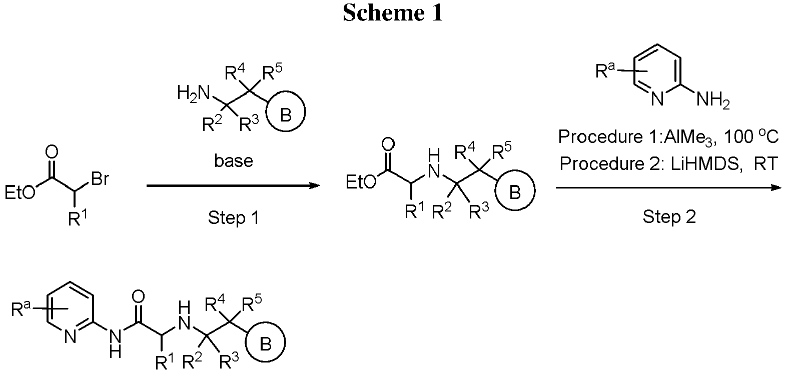

Scheme 1 illustrates a two-step synthetic sequence for the conversion of an α-bromoester to *N*-aryl-2-(alkylamino)acetamide. The method is useful for the synthesis of a subset of the compounds of Formula **I** where R¹ is a substituted phenyl and B, R^{a}, R², R³, R⁴, and R⁵ are as described herein.

### Method 1

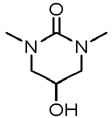

### 5-Hydroxy-1,3-dimethyltetrahydropyrimidin-2(1H)-one

### Method 1, step 1. 2-((Triisopropylsilyl)oxy)propane-1,3-diamine:

To a stirred solution of 1, 3-diaminopropan-2-ol (2.0 g, 22.19 mmol) in dry DCM (26 ml) was added triethylamine (6.74 g, 66.57 mmol) followed by the addition of TIPS-chloride (4.71 g, 24.41 mmol) drop-wise at 0 °C under an atmosphere of nitrogen. The reaction mixture was warmed to room temperature and stirred for 20 hours. After completion of reaction, the reaction mixture was quenched with saturated aqueous sodium bicarbonate solution (10 ml) and extracted with DCM (2 × 10 ml). The combined organic layers were dried over sodium sulfate and concentrated under reduced pressure to afford the title compound (5.0 g), which was used as such in next step without further purification. LCMS: *m*/*z* = 247.46 [M+1].

### Method 1, step 2. 5-((Triisopropylsilyl)oxy)tetrahydropyrimidin-2(1H)-one:

To a stirred solution of 2-((triisopropylsilyl)oxy)propane-1,3-diamine (5.0 g, 20.28 mmol) in methanol (81 ml) was added S, S'-dimethyldithiocarbonate (3.72 g, 30.43 mmol). Then the reaction was heated to 60 °C for 20 hours. After completion of reaction, the reaction mixture was concentrated under reduced pressure. The residue was purified by silica gel chromatography to afford the title compound (1.25 g, 21 %; 2 steps). LCMS: *m*/*z* = 273.48 [M+1].

### Method 1, step 3. 1,3-Dimethyl-5-((triisopropylsilyl)oxy)tetrahydropyrimidin-2(1H-one:

To a stirred solution of 5-((triisopropylsilyl)oxy)tetrahydropyrimidin-2(1*H*)-one (1.25 g, 4.59 mmol) in DMF (25 ml) was added NaH (0.551 g, 60% in oil; 13.77 mmol) at 0 °C. The resulting reaction mixture was stirred for 1 hour at 0 °C. Then methyl iodide (1.96 g, 13.77 mmol) was added to the reaction mixture. The reaction was warmed to room temperature and stirred for 20 hours. After completion of reaction, the reaction mixture was poured into ice cold water (25 ml) and extracted with ethyl acetate (2 × 25 ml). The organic layer was washed with water (2 × 25 ml), dried over sodium sulfate and concentrated under reduced pressure. The residue was purified by silica gel chromatography to afford the title compound (1.0 g, 73%). LCMS: *m*/*z* = 301.23 [M+1].

### Method 1, step 4. 5-Hydroxy-1,3-dimethyltetrahydropyrimidin-2(1H-one:

To a stirred solution of 1,3-dimethyl-5-((triisopropylsilyl)oxy)tetrahydropyrimidin-2(1*H*)-one (1.0 g, 3.33 mmol) in absolute ethanol (10 ml) was added 4*M* HCl in 1,4-dioxane (8.32 ml, 33.28 mmol) at 0 °C. Then the reaction mixture was stirred at room temperature for 5 hours. After completion of reaction, the reaction mixture was concentrated under reduced pressure. The solid was diluted with water (5 ml) and the aqueous layer was extracted with ethyl acetate (3 × 10 ml). The combined organic layer was washed with brine (10 ml), dried over anhydrous Na₂SO₄ and concentrated under reduced pressure to afford the title compound (1.5 g, used as such in next step without further purification). LCMS: *m*/*z* = 145.17 [M+1].

### Method 2

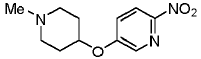

### 5-((1-Methylpiperidin-4-yl)oxy)-2-nitropyridine

### Method 2. 5-((1-Methylpiperidin-4-yl)oxy)-2-nitropyridine:

To a stirred solution of 1-methylpiperidin-4-ol (1.44 g, 12.49 mmol) and 5-fluoro-2-nitropyridine (0.7 g, 6.25 mmol) in dry DMF (7 ml) was added NaH (0.3 g, 95%, 12.49 mmol) portion wise at 0 °C. The reaction mixture was stirred at room temperature for 1 hour. The reaction mixture was then poured into ice cold water (10 ml) and the mixture was extracted with ethyl acetate (2 × 30 ml). The combined organic layers were washed with brine (10 ml), dried over anhydrous Na₂SO₄ and concentrated under reduced pressure to afford the title compound (0.54 g, 66 %). LCMS: *m*/*z* = 238.3 [M+1].

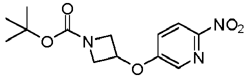

### tert-Butyl 3-((6-nitropyridin-3-yl)oxy)azetidine-1-carboxylate

### Method 2. tert-Butyl 3-((6-nitropyridin-3-yl)oxy)azetidine-1-carboxylate:

To a stirred solution of *tert*-butyl 3-hydroxyazetidine-1-carboxylate (3.00 g, 17.32 mmol) in dry THF (20 ml) was added NaH (1.38 g, 60% in oil, 34.64 mmol) at 0 °C. The reaction mixture was stirred at same temperature for 30 minutes and 5-fluoro-2-nitropyridine (2.04 g, 14.35 mmol) was added to the reaction mixture at 0 °C. After completion of the reaction, the reaction mixture was poured into ice cold water (75 ml) and extracted with ethyl acetate (2 × 100 ml). The combined organic layers were washed with brine (70 ml), dried over anhydrous sodium sulphate and concentrated under reduced pressure to afford the crude product. The residue was purified by silica gel chromatography to obtain the title compound (2.00 g, 47.1%). ¹H NMR (400 MHz, DMSO-d6) δ: 1.40 (s, 9H), 3.89 (d, J = 7.2 Hz, 2H), 4.37 (d, J = 7.6 Hz, 2H), 5.25 (s, 1H), 7.60-7.63 (m, 1H), 8.31 (dd, J = 8.8 Hz, J = 3.2 Hz, 2H).

### Method 3

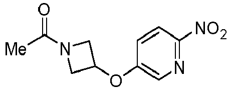

### 1-(3-((6-Nitropyridin-3-yl)oxy)azetidin- 1-yl)ethan- 1 -one

### Method 3, step 1. 5-(Azetidin-3-yloxy)-2-nitropyridine HCl:

To a stirred solution of tert-butyl 3-((6-nitropyridin-3-yl)oxy)azetidine-1-carboxylate (3.50 g, 11.85 mmol) in DCM (35 ml), 4M HCl in 1,4-Dioxane (3.5 ml, 14.0 mmol) was added drop wise at 0 °C. The reaction mixture was stirred at room temperature for 3 hours. After completion of the reaction, the reaction mixture was concentrated under reduced pressure. The resulting residue was triturated with n-pentane (15 ml) and filtered to afford the title compound (2.50 g, 91.0 %) as solid. LCMS: *m*/*z* = 196.04 [M+1].

### Method 3, step 2. 1-(3-((6-Nitropyridin-3-yl)oxy)azetidin-1-yl)ethan-1-one:

To a stirred solution of 5-(azetidin-3-yloxy)-2-nitropyridine hydrochloride (2.5 g, 10.79 mmol) in DCM (25 ml) was added TEA (4.3 g, 43.17 mmol) at 0 °C. Reaction mixture was stirred for 10 minutes at 0 °C. Then acetyl chloride (1.2 g, 15.19 mmol) was added drop wise at 0 °C. Reaction mixture was stirred at 0°C for another 1 hour. After completion of reaction, the reaction mixture was poured into saturated sodium bicarbonate solution (15 ml) and extracted with DCM (2 × 30 ml). The combined organic layers were washed with brine (20 ml), dried over anhydrous sodium sulphate and concentrated under reduced pressure. The resulting residue was purified by silica gel chromatography to afford the title compound (2.0 g, 78.1%). LCMS: *m*/*z* = 238.51 [M+1].

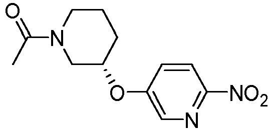

### (S)-1-(3-((6-Nitropyridin-3-yl)oxy)piperidin-1-yl)ethan-1-one

### Method 3, step 1. (S)-2-Nitro-5-(piperidin-3-yloxy)pyridine:

To a stirred solution of *tert*-butyl (S)-3-((6-nitropyridin-3-yl) oxy) piperidine-1-carboxylate (0.50 g, 1.54 mmol) in DCM (10 ml), TFA (2.5 ml, 5 V) was added drop wise at 0 °C. Reaction mixture was stirred at room temperature for 3 hours. After completion of reaction, the reaction mixture was poured into saturated sodium bicarbonate solution (15 ml) and extracted with DCM (2 × 30 ml). The combined organic layers were washed with brine (20 ml), dried over anhydrous sodium sulfate and concentrated under reduced pressure. The resulting residue was triturated with pentane (5 volumes) to afford the title compound (0.4 g, 70%) as solid. ¹H NMR (400 MHz, DMSO-d6): δ 8.31-8.33(m, 2H), 7.75-7.78 (m,1H), 4.53-4.59 (m, 1H), 3.10-3.13 (m, 1H), 2.75-2.80 (m, 1H), 2.57-2.62 (m, 2H), 2.05-2.09 (m, 1H), 1.68-1.71 (m, 1H), 1.51-1.68 (m, 1H), 1.46-1.50 (m, 1H); LCMS: *m*/*z* = 224.3 [M+1].

### Method 3, step 2. (S)-1-(3-((6-Nitropyridin-3-yl)oxy)piperidin-1-yl)ethan-1-one:

To a stirred solution of (*S*)-2-nitro-5-(piperidin-3-yloxy) pyridine (0.40 g, 1.79 mmol) in DCM (8 ml, 20 volumes) was added triethylamine (0.38 ml, 2.69 mmol) at 0 °C. The reaction mixture was stirred at 0 °C for 10 minutes and acetyl chloride (0.168 ml, 2.15 mmol) in DCM (1 ml) was added drop wise. The reaction mixture was stirred at 0 °C for 1 hour. The reaction mixture was poured into saturated sodium bicarbonate solution (15 ml) and extracted with DCM (2 × 30 ml). The combined organic layers were washed with brine (20 ml), dried over anhydrous sodium sulfate and concentrated under reduced pressure. The resulting residue was purified by silica gel chromatography to afford the title compound (0.3 g, 47%). LCMS: *m*/*z* = 266.4 [M+1].

### Method 4

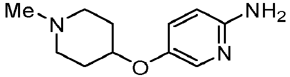

### 5-((1-Methylpiperidin-4-yl)oxy)pyridin-2-amine

### Method 4. 5-((1-Methylpiperidin-4-yl)oxy)pyridin-2-amine:

To a stirred solution of 5-((1-methylpiperidin-4-yl)oxy)-2-nitropyridine (0.54 g, 2.29 mmol) in a mixture of 1:1 methanol:ethyl acetate (12 ml) was added 10% Pd/C (0.054 g, 50% moisture). Then the reaction was stirred at room temperature under H₂ gas atmosphere for 3 hours. After completion of reaction, the reaction mixture was diluted with ethyl acetate and filtered through a pad of celite. The eluent was concentrated under reduced pressure to afford the title compound (0.22 g, 44 %) as a solid. ¹H NMR (400 MHz, DMSO-d6): 1.50-1.70 (m, 2H), 1.80-1.90 (m, 2H), 2.09-2.21 (m, 5H), 2.40-2.68 (m, 2H), 4.03-4.15 (m, 1H), 5.51 (s, 2H,-NH₂), 6.40 (d, *J*= 8.8 Hz, 1H), 7.10 (dd, *J =* 8.8 Hz, 2.8 Hz, 1H), 7.64 (d, *J* = 2.8 Hz, 1H).

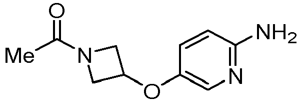

### Method 4. 1-(3-((6-Aminopyridin-3-yl)oxy)azetidin-1-yl)ethan-1-one:

To a stirred solution of 1-(3-((6-nitropyridin-3-yl)oxy)azetidin-1-yl)ethan-1-one (2.0 g, 8.43 mmol) in a mixture of 1: 1 ethanol:ethyl acetate (60 ml) was added Pt₂O (0.2 g, 10% w/w.). The reaction was stirred at room temperature under 15 Kg H₂ gas pressure for 16 hours. After completion of reaction, the reaction mixture was diluted with ethyl acetate (25 ml) and filtered through a pad of Celite. The celite pad was washed with ethyl acetate (2 × 25 ml) and the combined filtrate was concentrated under reduced pressure to afford the title compound (1.40 g, 80.1%) as a solid. LCMS: m/z = 208.05 [M+1].

### Method 5

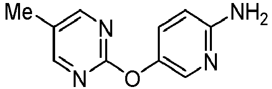

### 5-((5-Methylpyrimidin-2-yl)oxy)pyridin-2-amine

Method 5, step 1. 2-((6-Chloropyridin-3-yl)oxy)-5-methylpyrimidine: To a stirred solution of 6-chloropyridin-3-ol (1 g, 7.77 mmol) in NMP (5 ml), potassium tert-butoxide (1.3 g, 11.62 mmol) was added and the reaction mixture was stirred at room temperature for 1 hour. 2-chloro-5-methylpyrimidine (0.99 g, 7.77 mmol) was added and the reaction mixture was heated at 80 °C for 2 hours. After completion of the reaction, the reaction mixture was quenched with water (15 ml) and extracted with ethyl acetate (2 × 20 ml). The combined organic layers were washed with brine (10 ml), dried over anhydrous sodium sulfate and concentrated under reduced pressure to afford the crude product. The crude product was purified by silica gel chromatography to afford the title compound (1 g, 58%). LCMS: *m*/*z* = 222.35 [M+1].

### Method 5, step 2. N-(4-Methoxybenzyl)-5-((5-methylpyrimidin-2-yl)oxy)pyridin-2-amine:

To a stirred solution of 2-((6-chloropyridin-3-yl)oxy)-5-methylpyrimidine (0.5 g, 2.26 mmol) in 1,4-dioxane (10 ml) was added 4-methoxybenzylamine (0.37 g, 2.71 mmol), BINAP (0.07 g, 0.11 mmol), Pd₂(dba)₃ (0.04 g, 0.05 mmol) and sodium *tert*-butoxide (0.65 g, 6.78 mmol). The reaction mixture was purged with argon gas for 15 minutes and heated to 100 °C for 4 hours. After completion of the reaction, the reaction mixture was quenched with water (50 ml) and extracted with ethyl acetate (2 × 50 ml). The combined organic layers were washed with brine (10 ml), dried over anhydrous sodium sulphate and concentrated under reduced pressure to afford the crude product. The crude product was purified by silica gel chromatography to afford the title compound (0.15 g, 21%). LCMS: *mlz* = 323.43 [M+1].

### Method 5, step 3. 5-((5-Methylpyrimidin-2-yl)oxy)pyridin-2-amine:

To N-(4-methoxybenzyl)-5-((5-methylpyrimidin-2-yl)oxy)pyridin-2-amine (0.13 g, 0.40 mmol), TFA (1.3 ml, 10 V) was added drop wise at 0 °C. The reaction mixture was stirred at room temperature for 3 hours. After completion of reaction, the reaction mixture was concentrated under reduced pressure. The resulting residue was triturated with pentane (5 volumes) to afford the crude compound which was used in the next step without further purification (0.2 g, TFA salt). LCMS: *m*/*z* = 203.51 [M+1].

### Method 6

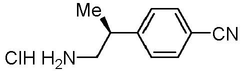

### (S)-4-(1-Aminopropan-2-yl)benzonitrile hydrochloride

### Method 6, step 1. Ethyl (E,Z)-3-(4-cyanophenyl)but-2-enoate:

To a stirred solution of potassium *tert*-butoxide (10.09 g, 89.7 mmol) in dry THF (90 ml) was added triethyl phosphonoacetate (20.08 g, 89.7 mmol) at 0 °C under an atmosphere of nitrogen. Then the reaction mixture was stirred for 15 minutes at the same temperature. The reaction was then warmed to room temperature and stirred for 1 hour. Then 4-acetylbenzonitrile (10.0 g, 69.0 mmol) was added as a solution in THF (50 ml) and the reaction was heated to 70 °C for 3 hours. After completion of reaction (monitored by TLC), the pH of the reaction mixture was adjusted to 3-4 with 1N HCl. The THF was removed under reduced pressure and the aqueous layer was extracted with ethyl acetate (2 × 50 ml). The combined organic layers were washed with brine (50 ml), dried over anhydrous Na₂SO₄ and concentrated under reduced pressure. The residue was purified by silica gel chromatography to afford the title compound (8.5 g, 58 %). ¹H NMR (400 MHz, DMSO-d6): 1.15 (t, *J* = 6.8 Hz, 1.5 H), 1.36 (t, *J* = 6.8 Hz, 3 H), 2.21 (s, 1.5 H), 2.60 (s, 3H), 4.05 (q, *J* = 7.1 Hz, 1H), 4.27 (q, *J* = 7.2 Hz, 2H), 6.01 (S, 0.5 H), 6.19 (S, 1H), 7.30-7.71 (m, 6 H).

### Method 6, step 2. Ethyl 3-(4-cyanophenyl)butanoate:

To a stirred solution of ethyl (*E, Z*) 3-(4-cyanophenyl)but-2-enoate (8.0 g, 37.2 mmol) in methanol: ethyl acetate (1:4, 140 ml) was added Pd/C (0.8 g, 10% w/w, 50% moisture). The reaction was stirred at room temperature under an atmosphere of hydrogen gas for 3 hours. The reaction mixture was diluted with ethyl acetate and filtered through a pad of celite. The combined organic layers were concentrated under reduced pressure to afford the title compound (4.5 g, 56%). ¹H NMR (400 MHz, CDCl₃): 1.23 (t, *J* = 7.2 Hz, 3H), 1.33 (d, *J* = 6.8 Hz, 3H), 2.62 (dd, *J* = 7.6 Hz,1.2 Hz, 2H), 3.70 (q, J = 7.2 Hz, 1H), 4.07-4.15 (m, 2 H), 7.37 (d, *J* = 8.0 Hz, 2H), 7.37 (d, *J* = 8.4 Hz, 2H).

### Method 6, step 3. 3-(4-Cyanophenyl)butanoic acid:

To a stirred solution of ethyl 3-(4-cyanophenyl)butanoate (4.5 g, 20.71 mmol) in a mixture of MeOH : THF : H₂O (4:2:1, 100 ml) was added LiOH (3.48 g, 82.95 mmol) at 5 °C to 10 °C. The resulting reaction mixture was stirred at room temperature for 1.5 hours. After completion of reaction (monitored by TLC), the reaction solvent was evaporated. The residue was dissolved in water (10 ml) and extracted with ethyl acetate (2 × 15 ml). The pH of the aqueous layer adjusted to 3-4 with concentrated HCl. The precipitate that formed was filtered off to afford title compound (3.8 g, 97 %) as a white solid. ¹H NMR (400 MHz, DMSO-d₆): 1.23 (d, *J* = 6.8, 3H), 2.58 (d, *J* = 7.6 Hz, 2H), 3.24 (q, *J* =7.2, 1H), 7.49 (d, *J* = 8.4 Hz, 2H), 7.77 (d, *J* = 8.4 Hz, 2H), 12.15 (s, 1H).

### Method 6, step 4. tert-Butyl (2-(4-cyanophenyl)propyl)carbamate:

To a stirred solution of 3-(4-cyanophenyl)butanoic acid (5.0 g, 26.45 mmol) in *tert*-butanol (65 ml) was added triethylamine (11.0 ml, 79.36 mmol) at room temperature. Then the reaction mixture was cooled to 5-10 °C and was added DPPA (12.30 g, 44.97 mmol) drop wise. After formation of acylazide, the reaction was stirred at 90 °C overnight. The reaction mixture was diluted with water (40 ml) and extracted with ethyl acetate (2 × 40 ml). The combined organic layers were washed with brine (25 ml), dried over anhydrous Na₂SO₄ and concentrated under reduced pressure. The resulting residue was purified by silica gel chromatography to afford the title compound (4.5 g, 66 %) as a solid. ¹H NMR (400 MHz, DMSO-d₆): 1.17 (d, *J* = 6.8 Hz, 2H), 1.33 (s, 9H), 2.90-3.00 (m, 1H), 3.04-3.15 (m, 2H), 6.91 (t, *J* = 5.2 Hz,1H, -NH), 7.42 (d, *J* = 8.4 Hz, 2H), 7.77 (d, *J* = 7.2 Hz, 2H).

### Method 6, step 5. 4-(1-Aminopropan-2-yl)benzonitrile hydrochloride:

To a stirred solution of *tert*-butyl-(2-(4-cyanophenyl)propyl)carbamate (4.5 g, 17.29 mmol) in methanol (9 ml) was added a solution of 4M HCl in dioxane (10.8 ml, 2.4 vol.) drop wise at 0 °C. The resulting mixture was stirred at room temperature for 2 hours. The reaction mixture was concentrated under reduced pressure to afford the title compound (2.81 g, 83 %) as a solid. ¹H NMR (400 MHz, DMSO-d₆): 1.28 (d, *J* = 6.8 Hz, 2H), 3.03 (d, *J* = 5.6 Hz, 2H), 3.15-3.26 (m, 1H), 7.55 (d, *J* = 8.0 Hz, 2 H), 7.83 (d, *J* = 8.0 Hz, 2H), 8.21 (s, 3H). LCMS: *m*/*z* = 161.6 [M+1].

### Method 6, step 6. 4-(1-Aminopropan-2-yl)benzonitrile:

4-(1-aminopropan-2-yl)benzonitrile hydrochloride was treated with an aqueous solution of saturated sodium bicarbonate and extracted with ethyl acetate (3 × 30 ml) to obtained the crude compound as liquid which was further purified by silica gel chromatography (DCM: MeOH = 90:10) to afford the racemic title compound a thick oil (2.29 g, 83%). ¹H NMR (400 MHz, CDCl₃): 1.28 (d, *J* = 6.8 Hz, 3H), 2.85 (d, *J* = 5.6 Hz, 3H), 7.34 (d, *J* = 7.2 Hz, 2 H), 7.63 (d, *J* = 7.2 Hz, 2H). LCMS: *m*/*z* =161.5 [M+1]. The racemic amine may be resolved into the enantiopure title compound by preparative chiral SFC using a CHIRALPAK AD-H column (250 mm, 50 mm, 5 microns; mobile phase 25% Acetonitrile:Methanol:Dimethylamine (80:20:0.1) in 75% CO₂). The early eluting isomer has been unambiguously assigned as (*S*)-4-(1-aminopropan-2-yl)benzonitrile by obtaining an x-ray co-crystal structures of related structurally-related inhibitors with a truncated form of p300.

### Method 7

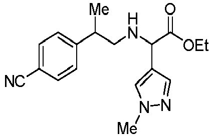

### Ethyl 2-((2-(4-cyanophenyl)propyl)amino)-2-(1-methyl-1H-pyrazol-4-yl)acetate

### Method 7, step 1. 2-((2-(4-Cyanophenyl)propyl)amino)-2-(1-methyl-1H-pyrazol-4-yl)acetic acid:

To a stirred solution of 4-(1-aminopropan-2-yl)benzonitrile hydrochloride (5 g, 30.86 mmol) in DCM (75 ml) were added TEA (3.12 g, 30.86 mmol), 2-oxoacetic acid (2.28 g, 30.86 mmol) and (1-methyl-1*H*-pyrazol-4-yl)boronic acid (3.80 g, 30.86 mmol) at room temperature. The reaction mixture was stirred at the same temperature for 15 minutes. After that HFIP (13.48 g, 80.24 mmol) was added and the reaction mixture was stirred for 16 hours at room temperature. The reaction was concentrated and the residue was stirred with DCM:pentane (3:7; 150 ml) for 30 minutes. A solid precipitated which was filtered on Büchner funnel and washed with n-pentane to afford title compound (5.5 g, 59 %). LCMS: *m*/*z* = 299 [M+1].

### Method 18, step 2. Ethyl 2-((2-(4-cyanophenyl)propyl)amino)-2-(1-methyl-1H-pyrazol-4-yl)acetate:

A mixture of 2-((2-(4-cyanophenyl)propyl)amino)-2-(1-methyl-1*H*-pyrazol-4-yl)acetic acid (5 g, 16.77 mmol) in DMF (100 ml) was heated at 80 °C until the reaction mixture became a clear solution. K₂CO₃ (5.79 g, 41.94 mmol) and ethyl iodide (2.61 g, 16.77 mmol) were added at the same temperature and the mixture was stirred for 30 minutes. The reaction mixture was then stirred at room temperature for 16 hours. The reaction was quenched with ice cold water (200 ml) and extracted with ethyl acetate (2 × 75 ml). The combined organic layers were washed with brine (100 ml), dried over anhydrous sodium sulfate, and concentrated under reduced pressure. The residue was purified by silica gel chromatography to afford the title compound (2.5 g, 45%) as a thick liquid. LCMS: *m*/*z* = 327.7 [M+1].

### Scheme 1

The starting materials required for the synthesis of examples prepared using Scheme 1 were either commercially available or prepared using methods 1 through 7.

### Example 1

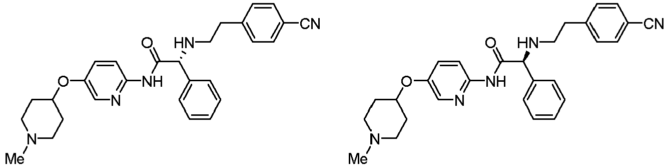

### (R)- and (S)-2-((4-cyanophenethyl)amino)-N-(5-((1-methylpiperidin-4-yl)oxy)pyridin-2-yl)-2-phenylacetamide

Scheme 1, step 1. Ethyl 2-((4-cyanophenethyl)amino)-2-phenylacetate: A mixture of ethyl 2-bromo-2-phenylacetate (2.0 g, 8.22 mmol), 4-(2-aminoethyl)benzonitrile hydrochloride (2.25 g, 12.33 mmol) and TEA (2.50 g, 24.66 mmol) in DMF (20 ml) was heated for 3 hours at 60 °C. The reaction mixture was poured into ice cold water (50 ml) and extracted with ethyl acetate (2 × 50 ml). The combined organic layers were washed with brine (25 ml), dried over anhydrous Na₂SO₄ and concentrated under reduced pressure. The resulting residue was purified by silica gel chromatography to afford the title compound (2.2 g, 86%) as a thick liquid. ¹H NMR (400 MHz, DMSO-d6): δ 1.10 (t, J = 7.2 Hz, 3H), 2.62-2.82 (m, 4H), 4.02-4.09 (m, 2H), 4.39 (d, J = 8.4 Hz, 1H), 7.28-7.35 (m, 5H), 7.40 (d, J = 8.4 Hz, 2H), 7.72 (d, J = 8.4 Hz, 2H). LCMS: *m*/*z* = 309.28 [M+1].

Scheme 1, step 2, procedure 2. (*R*)- and (*S*)-2-((4-cyanophenethyl)amino)-N-(5-((1-methylpiperidin-4-yl)oxy)pyridin-2-yl)-2-phenylacetamide: To a stirred solution of 5-((1-methylpiperidin-4-yl)oxy)pyridin-2-amine (0.10 g, 0.48 mmol), ethyl 2-((4-cyanophenethyl)amino)-2-phenylacetate (0.14 g, 0.48 mmol) in dry toluene (2 ml) was added trimethylaluminium (0.5 ml, 2M in toluene, 0.96 mmol) at 0 °C. The reaction mixture was stirred at 100 °C for 2 hours. After completion of the reaction, the reaction mixture was poured into ice cold water (25 ml) and extracted with ethyl acetate (2 × 50 ml). The combined organic layers were washed with brine (25 ml), dried over anhydrous sodium sulphate and concentrated under reduced pressure. The resulting residue was purified by silica gel chromatography to afford the title compound (0.10 g, 45%) as racemic mixture.

The racemic title compound was resolved by Chiral HPLC (CHIRALCEL OJ-H; 25% MeOH in liquid CO₂ + 0.1% DEA) to furnish the enantiopure compounds. The faster-eluting enantiomer (isomer 1) of the title compound was obtained as a solid. ¹H NMR (400 MHz, DMSO-d6): δ 1.60-1.65 (m, 2H), 1.89 (bs, 2H), 2.12-2.17 (m, 5H), 2.51 (bs, 2H), 2.74-2.77 (m, 2H), 2.85-2.86 (m, 2H), 4.36 (d, J = 4.0 Hz, 1H), 4.49 (d, J = 8.0 Hz, 1H), 7.26-7.36 (m, 3H), 7.42-7.45 (m, 5H), 7.74 (d, J = 8.4 Hz, 2H), 7.96 (d, J = 8.8 Hz, 1H), 8.02 (d, J = 2.8 Hz, 1H), 10.32 (s, 1H). LCMS: *m*/*z* = 470.51 [M+1].

The slower-eluting enantiomer (isomer 2) of the title compound was obtained as a solid. ¹H NMR (400 MHz, DMSO-d6): δ 1.58-1.66 (m, 2H), 1.89 (bs, 2H), 2.12-2.17 (m, 5H), 2.51 (bs, 2H), 2.75 (bs, 2H), 2.85-2.88 (m, 2H), 4.34-4.38 (m, 1H), 4.49 (d, J = 4.8 Hz, 1H), 7.25-7.35 (m, 3H), 7.42-7.45 (m, 5H), 7.74 (d, J = 8.0 Hz, 2H), 7.96 (d, J = 8.8 Hz, 1H), 8.02 (d, J = 2.8 Hz, 1H), 10.31 (s, 1H). LCMS: *m*/*z* = 470.51 [M+1].

### Example 2

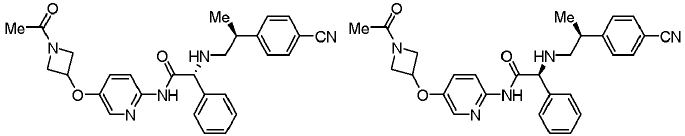

### Scheme 1, step 1. (R, S)- and (S, S)-ethyl 2-((2-(4-cyanophenyl)propyl)amino)-2-phenylacetate:

A mixture of ethyl 2-bromo-2-phenylacetate (9.11 g, 37.5 mmol), (S)-4-(1-aminopropan-2-yl)benzonitrile (5.0 g, 31.2 mmol) and TEA (13.1 ml, 93.7 mmol) in DMF (50 ml) was heated at 60 °C for 3 hours. The reaction mixture was poured into ice cold water (150 ml) and extracted with ethyl acetate (2 × 150 ml). The combined organic layers were washed with brine (150 ml), dried over anhydrous Na₂SO₄ and concentrated under reduced pressure. The resulting residue was purified by silica gel chromatography to afford a mixture of the title compounds (7.0 g, 70%) as a thick liquid. ¹H NMR (400 MHz, DMSO-d₆): 1.08 (t, *J* = 6.8 Hz, 3H), 1.16 (d, *J* = 6.8 Hz, 3H), 2.35-2.44 (m, 1H), 2.49-2.66 (m, 1H), 2.96 (q, *J* = 6.8 Hz, 1H), 3.96-4.06 (m, 2H), 4.32 (s, 1H), 7.26-7.42 (m, 7H), 7.74 (t, *J* = 7.6 Hz, 2H). LCMS: *m*/*z* = 323.6 [M+1].

### Scheme 1, step 2, procedure 1. (R)- and (S)-N-(5-((1-acetylazetidin-3-yl)oxy)pyridin-2-yl)-2-(((S)-2-(4-cyanophenyl)propyl)amino)-2-phenylacetamide:

To a stirred solution of 1-(3-((6-aminopyridin-3-yl)oxy)azetidin-1-yl)ethan-1-one (2.0 g, 9.65 mmol), ethyl 2-((2-(4-cyanophenyl)propyl)amino)-2-phenylacetate (3.7 g, 11.58 mmol) in DMF (20 ml) was added 1M LiHMDS in THF (24.2 ml, 24.12 mmol) at 0°C. The reaction mixture was stirred at 50°C for 1 hour. After completion of the reaction (monitored by TLC), the reaction mixture was poured into ice cold water (15 ml) and extracted with Ethyl acetate (2 × 25 ml). The combined organic layer was washed with brine (15 ml), dried over anhydrous Sodium sulphate and concentrated under reduced pressure. The resulting residue was purified by silica gel chromatography to afford the title compounds (3.0 g, 61%) as a mixture.

The title compounds were resolved by Chiral HPLC (CHIRALCEL OJ-H; 15% MeOH in LIQUID.CO₂ + 0.1% DEA) to furnish the enantiopure compounds. The faster-eluting enantiomer of the title compound was obtained as a solid (Isomer 1): ¹H NMR (400 MHz, DMSO-d₆): 1.23 (d, J = 6.8 Hz, 3H), 1.79 (s, 3H), 2.64 (d, J = 6.8 Hz, 3H), 3.00-3.04 (m, 1H), 3.74-3.77 (m, 1H), 4.07-4.10 (m, 1H), 4.25-4.29 (m, 1H), 4.44 (s, 1H), 4.51-4.55 (m, 1H), 5.05 (s, 1H), 7.24-7.45 (m, 8H), 7.75 (d, J = 8.0 Hz, 2H), 7.96-8.00 (m, 2H), 10.41 (s, 1H). LCMS: *m*/*z* = 484.5 [M+1]. The slower-eluting enantiomer of the title compound was obtained as a solid (Isomer 2): ¹H NMR (400 MHz, DMSO-d₆): 1.23 (d, J = 8.0 Hz, 3H), 1.79 (s, 3H), 2.68 (s, 3H), 3.02 (d, J = 6.0 Hz, 1H), 3.75-3.77 (m, 1H), 4.08-4.10 (m, 1H), 4.25-4.29 (m, 1H), 4.42 (s, 1H), 4.51-4.55 (m, 1H), 5.04 (s, 1H), 7.24-7.46 (m, 8H), 7.75 (d, J = 8 Hz, 2H), 7.94-7.98 (m, 2H), 10.22 (s, 1H). LCMS: *mlz* = 484.47 [M+1].

The following compounds were prepared using similar procedures to those described for Examples 1 and 2 using the appropriate starting materials.

The following compounds were prepared using similar procedures to those described for Example 1 and 2 using the appropriate starting materials. The separated isomers for each compound are listed in the order to which they elute. For example, in instances where there are two isomers, isomer 1 is the faster eluting isomer and isomer 2 is the slower-eluting isomer. In instances where there are four isomers, isomer 1 is the fastest eluting isomer followed by isomer 2, then isomer 3, and then isomer 4. Additionally, when more than one chiral column is listed the columns are used in sequential order as listed. For example, if two columns are listed for the purification of a compound with 2 stereocenters, the first column was used to resolve the mixture into pure stereoisomer 1, pure stereoisomer 2, and a mixture of stereoisomers 3 and 4 and the secondcolumn was used to resolve the mixture of stereoisomers 3 and 4.In some instances, a single chiral column may resolve all four stereoisomers.

The stereochemical representation (i.e., R or S) of each isomer of a compound is not drawn in the table and rather named to make clear that support for both is intended. Chiral carbon atom(s) are designated by the asterisk (*).

**Table 1.**

| *Example Nos.* | *Structure (Methods and Schemes for Preparation)* | *IUPAC Name* | *Exact Mass [M+1]* | *Chiral Column; Mobile Phase* |
|---|---|---|---|---|
| **3** | 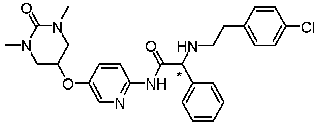 | (*S*)- and (*R*)-2-((4-chlorophenethyl)amino)-*N*-(5-((1,3-dimethyl-2-oxohexahydropyrimidin-5-yl)oxy)pyridin-2-yl)-2-phenylacetamide | Calc'd 508.2, Found 508.9 and 508.9 | CHIRALPAK IB; 35% (50:50 MeOH:IPA) in hexanes+ 0.1% DEA |
| Isomer 1 | | | | |
| Isomer 2 | | | | |
| **4** | 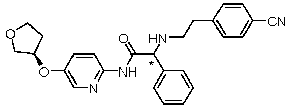 | (*S*)- and (*R*)-2-((4-cyanophenethyl)amino)-2-phenyl-*N*-(5-(((R)-tetrahydrofuran-3-yl)oxy)pyridin-2-yl)acetamide | Calc'd 443.2, Found 443.7 and 443.7 | CHIRALCEL OJ-H; 35% MeOH in Liquid CO₂ + 0.1% DEA |
| Isomer 1 | | | | |
| Isomer 2 | | | | |
| **5** | 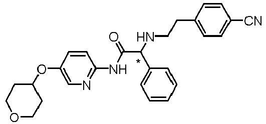 | (*S*)- and (*R*)-2-((4-cyanophenethyl)amino)-2-phenyl-*N*-(5-((tetrahydro-2H-pyran-4-yl)oxy)pyridin-2-yl)acetamide | Calc'd 457.2, Found 458.0 and 458.0 | CHIRALCEL OX-H; 35% (50:50 MeOH:IPA) in hexanes + 0.1% DEA |
| Isomer 1 | | | | |
| Isomer 2 | | | | |
| **6** | 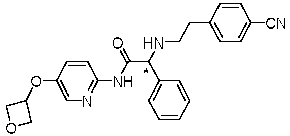 | (*S*)- and (*R*)-2-((4-cyanophenethyl)amino)-*N*-(5-(oxetan-3-yloxy)pyridin-2-yl)-2-phenylacetamide | Calc'd 429.2, Found 429.4 and 429.4 | CHIRALCEL OX-H; 35% (50:50 MeOH:IPA) in hexanes + 0.1% DEA |
| Isomer 1 | | | | |
| Isomer 2 | | | | |
| **7** | 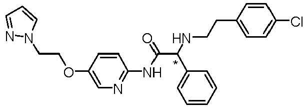 | (*S*)- and (*R*)-*N-*(5-(2-(1H-pyrazol-1-yl)ethoxy)pyridin-2-yl)-2-((4-chlorophenethyl)amino)-2-phenylacetamide | Calc'd 476.2, Found 476.5 and 476.5 | CHIRALPAK IB; 35% (50:50 IPA:MeOH) in hexanes + 0.1% DEA |
| Isomer 1 | | | | |
| Isomer 2 | | | | |
| **8** | 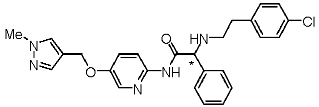 | (*S*)- and (*R*)-2-((4-chlorophenethyl)amino)-*N*-(5-((1-methyl-1H-pyrazol-4-yl)methoxy)pyridin-2-yl)-2-phenylacetamide | Calc'd 476.2, Found 476.3 and 476.3 | CHIRALPAK IC; 25% (50:50 MeOH:IPA) in Liquid CO₂ + 0.1% DEA |
| Isomer 1 | | | | |
| Isomer 2 | | | | |
| **9** | 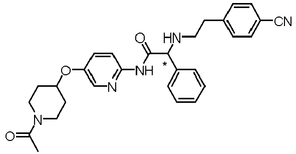 | (*S*)- and (*R*)-*N*-(5-((1-acetylpiperidin-4-yl)oxy)pyridin-2-yl)-2-((4-cyanophenethyl)amino)-2-phenylacetamide | Calc'd 498.2, Found 498.7 and 498.7 | CHIRALCEL OX-H; 28% (50:50 IPA:MeOH) in Liquid CO₂ + 0.1% DEA |
| Isomer 1 | | | | |
| Isomer 2 | | | | |
| **10** | 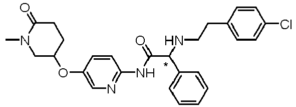 | (*S*, *S)-,* (*S*, *R*)-, (*R*, *R*)- and (*R*, *S*)- 2-((4-chlorophenethyl)amino)-*N*-(5-((1-methyl-6-oxopiperidin-3-yl)oxy)pyridin-2-yl)-2-phenylacetamide | Calc'd 493.2, Found 493.3, 493.5 , 493.5 and 493.5 | 1-CHIRALPAK IB; 30% (50:50 IPA:MeOH) in hexanes + 0.1% DEA 2-CHIRALCEL OX-H; 30% MeOH in Liquid CO₂+ 0.1% DEA |
| Isomer 1 | | | | |
| Isomer 2 | | | | |
| Isomer 3 | | | | |
| Isomer 4 | | | | |
| **11** | 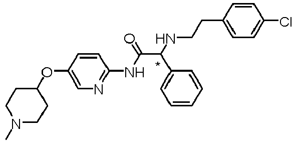 | (*S*)- and (*R*)-2-((4-chlorophenethyl)amino)-*N*-(5-((1-methylpiperidin-4-yl)oxy)pyridin-2-yl)-2-phenylacetamide | Calc'd 479.2, Found 479.4 and 479.6 | CHIRALCEL OX-H; 30% (50:50 IPA:MeOH) in hexanes + 0.1% DEA |
| Isomer 1 | | | | |
| Isomer 2 | | | | |
| **12** | 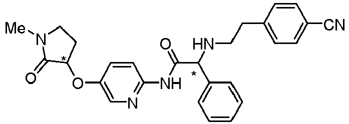 | (*S*, *S*)-, (*S*, *R*)-, (*R*, *R*)- and (*R*, *S*)- 2-((4-cyanophenEthyl)amino)-N-(5-((1-methyl-2-oxopyrrolidin-3-yl)oxy)pyridin-2-yl)-2-phenylacetamide | Calc'd 470.2, Found 470.7, 470.7, 470.3 and 470.7 | 1-CHIRALCEL OJ-H; 12% MeOH in Liquid CO₂ + 0.1% DEA 2-CHIRALPAK IB; 35% (50:50 IPA:MeOH) in hexanes + 0.1% DEA |
| Isomer 1 | | | | |
| Isomer 2 | | | | |
| Isomer 3 | | | | |
| Isomer 4 | | | | |
| **13** | 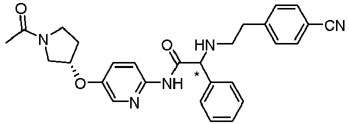 | (*S*, *S*)-, (*S*, *R*)-N-(5-((-1-acetylpyrrolidin-3-yl)oxy)pyridin-2-yl)-2-((4-cyanophenEthyl)amino)-2-phenylacetamide | Calc'd 484.2, Found 484.7 and 484.7 | CHIRALPAK IC; 35% (70:30 IPA:ACN) in hexanes + 0.1% DEA |
| Isomer 1 | | | | |
| Isomer 2 | | | | |
| **14** | 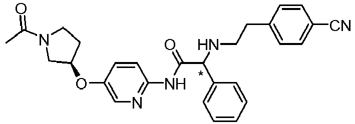 | (*R*, *R*)- and (*R*, *S*)-N-(5-((-1-acetylpyrrolidin-3-yl)oxy)pyridin-2-yl)-2-((4-cyanophenEthyl)amino)-2-phenylacetamide | Calc'd 484.2, Found 484.7 and 484.8 | CHIRALCEL OJ-H; 20% MeOH in Liquid CO₂+ 0.1% DEA |
| Isomer 1 | | | | |
| Isomer 2 | | | | |
| **15** | 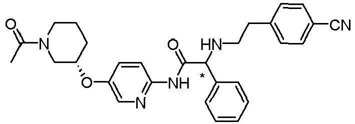 | (*S*)- and (*R*)- *N*-(5-(((S)-1-acetylpiperidin-3-yl)oxy)pyridin-2-yl)-2-((4-cyanophenEthyl)amino)-2-phenylacetamide | Calc'd 498.2, Found 498.5 and 498.5 | CHIRALCEL OJ-H; 35% MeOH in Liquid CO₂+ 0.1% DEA |
| Isomer 1 | | | | |
| Isomer 2 | | | | |
| **16** | 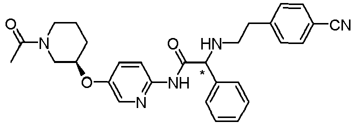 | (*S*)- and (*R*)- *N*-(5-(((R)-1-acetylpiperidin-3-yl)oxy)pyridin-2-yl)-2-((4-cyanophenEthyl)amino)-2-phenylacetamide | Calc'd 498.2, Found 498.8 and 498.7 | CHIRALCEL OJ-H; 35% MeOH in Liquid CO₂+ 0.1% DEA |
| Isomer 1 | | | | |
| Isomer 2 | | | | |
| **17** | 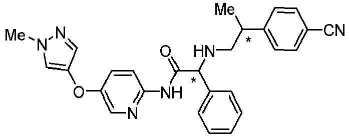 | *(S*, *S*)-*,* (*S*, *R*)-, (*R*, *R*)- and (*R*, *S*)- 2-((2-(4-cyanophenyl)propyl)amin o)-N-(5-((1-methyl-1H-pyrazol-4-yl)oxy)pyridin-2-yl)-2-phenylacetamide | Calc'd 467.2, Found 467.8, 467.8, 467.8 and 467.8 | 1-CHIRALCEL OJ-H; 25% MeOH in Liquid CO₂+ 0.1% DEA 2-CHIRALCEL OJ-H; 30% MeOH in Liquid CO₂+ 0.1% DEA |
| Isomer 1 | | | | |
| Isomer 2 | | | | |
| Isomer 3 | | | | |
| Isomer 4 | | | | |
| **18** | 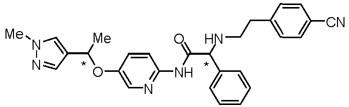 | (*S*, *S*)-, (*S*, *R*)-, (*R*, *R*)- and (*R*, *S*)- 2-((4-cyanophenethyl)amino)-N-(5-(1-(1-methyl-1H-pyrazol-4-yl)ethoxy)pyridin-2-yl)-2-phenylacetamide | Calc'd 481.2, Found 481.4, 481.5, 481.4 and 481.5 | 1-CHIRALPAK IC; 30% (50:50 IPA:ACN) in Liquid CO₂ + 0.1% DEA 2-CHIRALPAK IC; 25% (50:50 IPA:ACN) in Liquid CO₂ + 0.1% DEA |
| Isomer 1 | | | | |
| Isomer 2 | | | | |
| Isomer 3 | | | | |
| Isomer 4 | | | | |
| **19** | 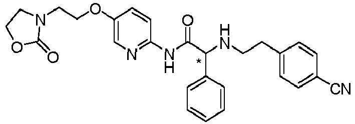 | (*S*)- and (*R*)- 2-((4-cyanophenEthyl)amino)-N-(5-(2-(2-oxooxazolidin-3-yl)ethoxy)pyridin-2-yl)-2-phenylacetamide | Calc'd 486.2, Found 486.8 and 486.8 | CHIRALCEL OX-H; 40% (70:30 IPA:ACN) in hexanes + 0.1% DEA |
| Isomer 1 | | | | |
| Isomer 2 | | | | |
| **20** | 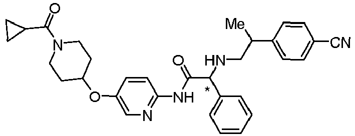 | (*S*, *S*)- and (*R*, *S*)-2-((2-(4-cyanophenyl)propyl)amin o)-N-(5-((1-(cyclopropanecarbonyl)pi peridin-4-yl)oxy)pyridin-2-yl)-2-phenylacetamide | Calc'd 538.2, Found 539.1 and 539.1 | CHIRALCEL OJ-H; 30% MeOH in Liquid CO₂+ 0.1% DEA |
| Isomer 1 | | | | |
| Isomer 2 | | | | |
| **21** | 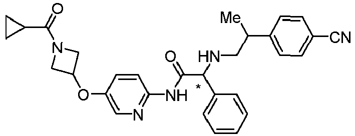 | (*S*, *S*)- and (*R*, *S*)-2-((2-(4-cyanophenyl)propyl)amin o)-N-(5-((1-(cyclopropanecarbonyl)azetidin-3-yl)oxy)pyridin-2-yl)-2-phenylacetamide | Calc'd 510.2, Found 510.7 and 510.8 | CHIRALCEL OX-H; 35% (70:30 IPA:ACN) in hexanes + 0.1% DEA |
| Isomer 1 | | | | |
| Isomer 2 | | | | |
| **22** | 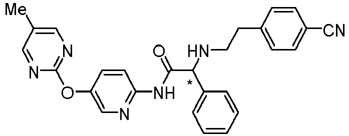 | (*S*)- and (*R*)- 2-((4-cyanophenEthyl)amino)-N-(5-((5-methylpyrimidin-2-yl)oxy)pyridin-2-yl)-2-phenylacetamide | Calc'd 465.2, Found 465.5 and 465.5 | CHIRALCEL OJ-H; 20% MeOH in Liquid CO₂+ 0.1% DEA |
| Isomer 1 | | | | |
| Isomer 2 | | | | |
| **23** | 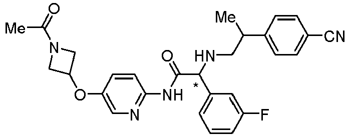 | (*S*, *S*)- and (*R*, *S*)-N-(5-((1-acetylazetidin-3-yl)oxy)pyridin-2-yl)-2-((2-(4-cyanophenyl)-propyl)amino)-2-(3-fluorophenyl)acetamide | Calc'd 502.2, Found 502.9 and 502.9 | CHIRALPAK AD-H; 20% IPA in Liquid CO₂ + 0.1% DEA |
| Isomer 1 | | | | |
| Isomer 2 | | | | |
| **24** | 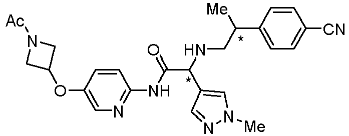 | (*S*, *S*)- and (*R*, *S*)-*N*-(5-((1-acetylazetidin-3-yl)oxy)pyridin-2-yl)-2-((2-(4-cyanophenyl)-propyl)amino)-2-(1-methyl-1H-pyrazol-4-yl)acetamide | Calc'd 488.2, Found 488.7, 488.6, 488.7 and 488.7 | 1-CHIRALPAK AD-H; 25% MeOH in Liquid CO₂ + 0.1% DEA 2-CHIRALPAK AD-H; 35% (50:50 IPA:MeOH) in Liquid CO₂ + 0.1% DEA |
| Isomer 1 | | | | |
| Isomer 2 | | | | |
| **25** | 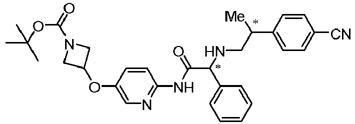 | tert-butyl 3-((6-(2-((2-(4-cyanophenyl)propyl)amin o)-2-phenylacetamido)pyridin-3-yl)oxy)azetidine-1 - carboxylate | Calc'd 542.27, Found 542.5 and | CHIRALPAK AD-H; 50% MeOH in acetonitrile + 0.1% DEA |
| Isomer 1 | | | | |
| Isomer 2 | | | | |

### Example 26

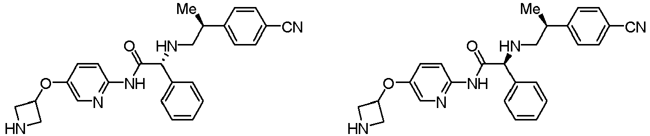

### (R, S)- and (S, S)-N-(5-(azetidin-3-yloxy)pyridin-2-yl)-2-(((S)-2-(4-cyanophenyl)propyl)amino)-2-phenylacetamide

Step 1. (*R, S)-* and (*S, S*)- *N*-(5-(azetidin-3-yloxy)pyridin-2-yl)-2-(((S)-2-(4-cyanophenyl)propyl)amino)-2-phenylacetamide: To a stirred solution of tert-butyl 3-((6-(2-((2-(4-cyanophenyl)propyl)amino)-2-phenylacetamido)pyridin-3-yl)oxy)azetidine-1-carboxylate (0.50 g, 0.92 mmol) in DCM (15 ml), TFA (2.5 ml) was added drop wise at 0 °C. Reaction mixture was stirred at room temperature for 3 hours. After completion of the reaction, reaction mixture was poured into saturated aq. Sodium bicarbonate solution (15 ml) and extracted with DCM (2 × 30 ml). The combined organic layer was washed with brine (20 ml), dried over anhydrous sodium sulphate and concentrated under reduced pressure. The resulting residue was triturated with pentane (50 ml) and obtained solid was purified by reverse phase chromatography to afford the racemic title compound (0.275 g, 68%).

The racemic title compound was resolved by chiral HPLC (CHIRALCEL AD-H; 30% (50:50 MeOH:ACN) in liquid CO₂ + 0.1% DEA) to furnish the enantiopure compounds. The faster-eluting enantiomer of the title compound was obtained as a solid (Isomer 1): ¹H NMR (400 MHz, DMSO-d6): δ 1.19 (d, J = 6.4 Hz, 3H), 2.65 (d, J = 8.8 Hz, 2H), 3.00-3.02 (m, 1H), 3.51 (s, 2H), 3.79 (bs, 2H), 4.39 (s, 1H), 4.99 (d, J = 5.6 Hz, 1H), 7.25-7.45 (m, 8H), 7.73 (d, J = 7.6 Hz, 2H), 7.88-7.93 (m, 2H), 10.15 (s, 1H). LCMS: m/z = 442.46 [M+1]. The slower-eluting enantiomer of the title compound was obtained as a solid (Isomer 2): ¹H NMR (400 MHz, DMSO-d6): δ 1.21 (d, J = 6.8 Hz, 3H), 2.62 (d, J = 6.8 Hz, 2H), 3.00-3.01 (m, 1H), 3.66 (s, 2H), 3.98 (bs, 2H), 4.42 (s, 1H), 5.01 (s, 1H), 7.24-7.44 (m, 8H), 7.74 (d, J = 8.0 Hz, 2H), 7.91-7.96 (m, 2H), 10.37 (s, 1H). LCMS: m/z = 442.35 [M+1].

### Example 27

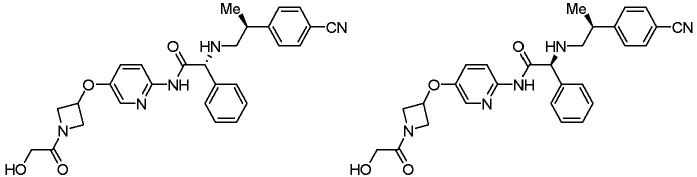

### (R, S)- and (S, S)-2-(((S)-2-(4-cyanophenyl)propyl)amino)-N-(5-((1-(2-hydroxyacetyl)azetidin-3-yl)oxy)pyridin-2-yl)-2-phenylacetamide

### Step 1. (R, S)- and (S, S)-2-(((S)-2-(4-cyanophenyl)propyl)amino)-N-(5-((1-(2-hydroxyacetyl)azetidin-3-yl)oxy)pyridin-2-yl)-2-phenylacetamide:

To a stirred solution of glycolic Acid (0.058 g, 0.76 mmol) in dry DMF (1.5 ml) was added HATU (0.35 g, 0.92 mmol) at room temperature under nitrogen atmosphere. The above reaction mixture was stirred at room temperature for 1 hour. After 1 hour, to the reaction mixture TFA salt of N-(5-(azetidin-3-yloxy)pyridin-2-yl)-2-((2-(4-cyanophenyl)propyl)amino)-2-phenylacetamide (0.3 g, 0.54 mmol) in DMF (1.5 ml) was added followed by DIPEA (0.21 g, 1.62 mmol). The reaction mixture was stirred at room temperature for 16 hours. After completion of the reaction (monitored by the TLC), reaction mixture was quenched with ice water and extracted with EtOAc (2 × 10 ml). The combined organic layers were washed with brine (20 ml), dried over anhydrous sodium sulfate and concentrated under reduced pressure to obtain the crude product. The crude product was purified by Combi-flash chromatography in DCM:MeOH followed by reverse phase HPLC to afford the racemic title compound (0.105 g, 39%).

The racemic title compound was resolved by chiral HPLC (CHIRALCEL OJ-H; 20% (50:50 IPA:ACN) in liquid CO₂ + 0.1% DEA) to furnish the enantiopure compounds. The faster-eluting enantiomer of the title compound was obtained as a solid (Isomer 1): Isomer-1: ¹H NMR (400 MHz, DMSO-d6): δ 1.22 (d, J = 6.8 Hz, 3H), 2.69 (s, 3H), 3.03 (d, J = 6.4 Hz, 1H), 3.83 (d, J = 8.8 Hz, 1H), 3.94 (d, J = 6.0 Hz, 2H), 4.15 (d, J = 7.2 Hz, 1H), 4.34 (t, J = 6.4 Hz, 1H), 4.43 (s, 1H), 4.62 (t, J = 6.4 Hz, 1H), 5.03 (t, J = 6.0 Hz, 1H), 5.09 (s, 1H), 7.25-7.47 (m, 6H), 7.76 (d, J = 8.0 Hz, 2H), 7.97 (d, J = 10.8 Hz, 2H), 10.22 (s, 1H). LCMS: m/z = 500.5 [M+1]. Isomer-2: ¹H NMR (400 MHz, DMSO-d6): δ 1.23 (d, J = 6.4 Hz, 3H), 2.65 (s, 3H), 3.02 (d, J = 6.8 Hz, 1H), 3.82 (d, J = 10.4 Hz, 1H), 3.93 (d, J = 5.6 Hz, 2H), 4.10-4.15 (m, 1H), 4.33-4.43 (m, 2H), 4.61 (m, 1H), 5.00 (t, J = 6.4 Hz, 1H), 5.08 (s, 1H), 7.26-7.45 (m, 6H), 7.75 (d, J = 7.6 Hz, 2H), 7.98 (d, J = 13.2 Hz, 2H), 10.39 (s, 1H). LCMS: m/z = 500.8 [M+1].

### Biochemical and Cellular Assays

The activity of the compounds described herein as p300/CBP HAT inhibitors may be readily determined using a scintillation proximity assay (SPA) methodology (Udenfriend, S.; Gerber, L.; Nelson, N. Scintillation Proximity Assay: A Sensitive and Continuous Isotopic Method for Monitoring Ligand/Receptor and Antigen/Antibody Interactions. *Anal. Biochem.* **1987,** *161*, 494-500). In particular, the compounds of the following examples had activity in reference assays by exhibiting the ability to inhibit the acetylation of histone peptide by a truncated form of the p300 enzyme (p300 HAT). Any compound exhibiting a IC₅₀ of about 100 µM or below would be considered a p300/CBP-HAT inhibitor as defined herein.

In a typical experiment the p300 HAT inhibitory activity of the compounds described herein was determined in accordance with the following experimental method.

The p300 HAT domain (residues 1287-1666) was expressed and purified with an N-terminal His tag from *Escherichia coli* cells. The expressed protein was purified by Ni2+ affinity, followed by anion exchange chromatography. Appropriate fractions were pooled and buffer exchanged into 20mM Hepes pH 7.5, 150mM NaCl, and 1mM TCEP.

Compounds of interest solubilized in DMSO were stamped in a Greiner black 384-well plate in a 10-point duplicate dose response using an Echo 550 (Labcyte). p300-HAT domain purified in-house (aa 1287-1666) was diluted to 6nM in reaction buffer (50mM Tris pH 8.0, 100mM NaCl, 1mM DTT, 0.069mM Brij-35, 0.1mM EDTA, 0.1mg/mL BSA), combined with 4.14µM AcCoA (Sigma-Aldrich) and 0.46µM ³H-AcCoA (PerkinElmer), and 12.5µL added to each well and incubated for 30min at RT. Reactions were initiated with 12.5µL 2µM biotinylated H3(1-21) peptide (New England Peptide) and run for 1hr at RT, then quenched with 20µL stop solution (200mM Tris pH 8.0, 200mM EDTA, 2M NaCl, 160µM anacardic acid). 35µL of the reaction volume was transferred to a 384-well streptavidin FlashPlate (PerkinElmer) using a Bravo liquid handler (Velocity 11) and incubated for 1.5hr at RT. Plates were aspirated, washed with 95µL wash buffer (15mM Tris pH 8.5, 0.069µM Brij-35), aspirated, sealed, and scintillation counts read on a Topcount (PerkinElmer). Data were analyzed in Genedata to determine inhibitor IC₅₀ values.

The full length p300 SPA assay was run following the same protocol as p300 HAT SPA assay, but used 6nM purified full length p300 (purchased from Active Motif) in place of the purified p300-HAT domain.

Select compounds were also evaluated in a H3K18Ac MSD cellular assay that measures the ability of compounds to inhibit the the acetylation of chromatin at H3K18, a process catalyzed by p300 and CBP. In a typical experiment the p300 HAT inhibitory activity inside cells of the compounds described herein was determined in accordance with the following experimental method. 20k HCT-116 cells per well are plated in 75µL RPMI+10% FBS media the night before treatment. Compounds plated in DMSO at 4x final concentration are resuspended in 30µL RPMI+10% FBS, then 25µL is combined with corresponding wells containing cells. Treated cells are incubated for 2hr at 37°C, then lysed in 500µL final volume and frozen at -80°C. MSD plates (Meso Scale Discovery) are coated overnight at 4°C with 60µL 1:500 α-total histone antibody (Millipore MAB3422) in PBS. Plates are then blocked with 5% BSA in TBST shaking at RT for 1hr, washed, and 30µL lysate added to each well for 2hr shaking at RT. Plates are washed and 25µL 1:216 α-H3K18ac antibody (CST 9675) in PBS added, then incubated for 1hr shaking at RT. Plates are washed again, then 25µL 1:1000 Sulfo-Tag goat α-rabbit antibody (Meso Scale Discovery R32Ab-1) in PBS is added for 1hr shaking at RT. Plates are washed once more, then 150µL 1× Read Buffer (MSD #R92TD-3) is added to all wells and read on MSD SECTOR Imager 2400 using the conventional read setup.

The compounds of the following examples had activity in inhibiting the HAT domain of the p300 enzyme in the aforementioned assays with a IC₅₀ of less than about 100 µM. Many of compounds described herein had activity in inhibiting the HAT domain of the p300 enzyme in the aforementioned assays, with an IC₅₀ of less than about 10 µM, preferably less than or about 0.1 µM. Additional data is provided in the following Examples. Such a result is indicative of the intrinsic activity of the compounds in use as inhibitors of the histone acetyl transferase domain of the p300 enzyme. In general, one of ordinary skill in the art would appreciate that a substance is considered to effectively inhibit p300 HAT activity if it has a IC₅₀ of less than or about 1 µM, preferably less than or about 0.1 µM. The present disclosure also includes compounds which possess activity as inhibitors of other histone acetyl transferase enzymes, such as CBP-HAT. The p300 HAT IC₅₀ is a measure of the ability of the test compound to inhibit the action of the p300 enzyme.

P300 inhibitory activity of compounds described herein estimated from a P300 HAT SPA assay are shown by Table 2. All activities are the average of at least 2 replicate titrations.

**Table 2**

| ***Example number*** | ***Isomer*** | ***P300 HAT SPA IC₅₀ (*µ*M)*** | ***FL P300 IC₅₀ (µM)*** | ***H3K18Ac MSD EC₅₀ (µM)*** |
|---|---|---|---|---|
| **1** | Isomer 1 | 0.0277 | <0.002 | 0.035 |
| | Isomer 2 | >5 | 0.24 | |
| **2** | Isomer 1 | 0.0212 | <0.002 | 0.020 |
| | Isomer 2 | >2 | | |
| **3** | Isomer 1 | >2 | | |
| | Isomer 2 | 0.117 | | |
| **4** | Isomer 1 | 0.0914 | | |
| | Isomer 2 | >2 | | |
| **5** | Isomer 1 | 0.0976 | 0.00369 | |
| | Isomer 2 | >2 | 0.137 | |
| **6** | Isomer 1 | 0.0399 | | |
| | Isomer 2 | 1.14 | | |
| **7** | Isomer 1 | >2 | | |
| | Isomer 2 | 0.0985 | | |
| **8** | Isomer 1 | 1.36 | | |
| | Isomer 2 | 0.0168 | | 0.069 |
| **9** | Isomer 1 | 0.0564 | 0.00421 | |
| | Isomer 2 | >2 | 0.102 | |
| **10** | Isomer 1 | 0.0942 | | |
| | Isomer 2 | >2 | | |
| | Isomer 3 | 0.0454 | | |
| | Isomer 4 | >2 | | |
| **11** | Isomer 1 | 0.0266 | | |
| | Isomer 2 | >2 | | |
| **12** | Isomer 1 | 0.065 | | |
| | Isomer 2 | 0.063 | | |
| | Isomer 3 | >2 | | |
| | Isomer 4 | >2 | | |
| **13** | Isomer 1 | >2 | | |
| | Isomer 2 | 0.0599 | | |
| **14** | Isomer 1 | 0.0362 | | |
| | Isomer 2 | >2 | | |
| **15** | Isomer 1 | 0.272 | | |
| | Isomer 2 | >2 | | |
| **16** | Isomer 1 | 0.164 | | |
| | Isomer 2 | >2 | | |
| **17** | Isomer 1 | 0.0337 | | |
| | Isomer 2 | >2 | | |
| | Isomer 3 | >2 | | |
| | Isomer 4 | >2 | | |
| **18** | Isomer 1 | 0.311 | | |
| | Isomer 2 | 0.209 | | |
| | Isomer 3 | >2 | | |
| | Isomer 4 | >2 | | |
| **19** | Isomer 1 | 0.0566 | | |
| | Isomer 2 | >2 | | |
| **20** | Isomer 1 | >2 | | |
| | Isomer 2 | 0.0267 | | |
| **21** | Isomer 1 | 0.0274 | <0.002 | |
| | Isomer 2 | >2 | | |
| **22** | Isomer 1 | >2 | | |
| | Isomer 2 | 1.39 | | |
| **23** | Isomer 1 | 0.0233 | <0.003 | 0.0319 |
| | Isomer 2 | 0.965 | | |
| **24** | Isomer 1 | 0.038 | <0.003 | 0.057 |
| | Isomer 2 | 0.578 | | |
| | Isomer 3 | >2 | | |
| | Isomer 4 | >2 | | |
| **25** | Isomer 1 | >1 | | |
| | Isomer 2 | 0.115 | | |
| **26** | Isomer 1 | 0.65 | | |
| | Isomer 2 | 0.014 | 0.00149 | 0.182 |
| **27** | Isomer 1 | 0.247 | | |
| | Isomer 2 | >1 | | |

While we have described a number of embodiments, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of example.

Unless otherwise defined, all technical and scientific terms used herein are accorded the meaning commonly known to one with ordinary skill in the art.

## Claims

1. A compound having the Formula I: 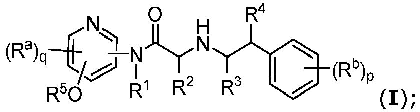 or a pharmaceutically acceptable salt thereof, wherein
R¹, R³, and R⁴ are each independently hydrogen or C₁₋₄alkyl;
R² is phenyl or pyrazolyl, each of which are optionally substituted with halo or C₁₋₄alkyl;
R⁵ is a C₁₋₆alkyl substituted with 4- to 6-membered heterocyclyl or 5- to 6-membered heteroaryl, wherein each of said 4- to 6-membered heterocyclyl or 5- to 6-membered heteroaryl are optionally substituted with 1 to 3 groups selected from R^{d}; or R⁵ is 4- to 6-membered heterocyclyl or 5- to 6-membered heteroaryl, wherein each of said 4- to 6-membered heterocyclyl or 5- to 6-membered heteroaryl are optionally substituted with 1 to 3 groups selected from R^{d};
R^{a}, if present, is C₁₋₃alkyl, C₁₋₃alkoxy, halo(C₁₋₃alkyl), haloC₁₋₃alkoxy, or halo;
R^{b} is halo, cyano, or -SO₂NH₂;
each R^{d} is independently halo, CN, oxo, NO₂, C₁₋₆alkyl, C₂₋₆alkenyl, C₁₋₆alkoxy, halo(C₁₋₆alkoxy), halo(C₁₋₆alkyl), -C₁₋₆alkylOR^{e}, -C(O)R^{f}, -C(O)OR, -C₁₋₆alkylC(O)OR^{e}, -C(O)N(R^{e})₂, -C(O)NR^{e}C₁₋₆alkylOR^{e}, -OC₁₋₆alkylN(R^{e})₂, -C₁₋₆alkylC(O)N(R^{e})₂, -C₁₋₆alkylN(R^{e})₂, -N(R^{e})₂, -C(O)NR^{e}C₁₋₆alkylN(R^{e})₂, -NR^{e}C₁₋₆alkylN(R^{e})₂, -NR^{e}C₁₋₆alkylOR^{e}, -SOR^{e}, -S(O)₂R^{e}, -SON(R^{e})₂, -SO₂N(R^{e})₂, -O(C₃-C₆)cycloalkyl, -O-C₁₋₄alkylaryl, -C₁₋₆alkyl(C₃-C₆)cycloalkyl, -C₁₋₆alkylaryl, -C₁₋₆alkylheteroaryl, -C₁₋₆alkylheterocyclyl, (C₃-C₆)cycloalkyl, heterocyclyl, heteroaryl, or aryl, wherein each of said (C₃-C₆)cycloalkyl, heterocyclyl, aryl, and heteroaryl alone and in connection with -O(C₃-C₆)cycloalkyl, -C₁₋₆alkyl(C₃-C₆)cycloalkyl, -C₁₋₆alkylaryl, -C₁₋₆alkylheteroaryl, and -C₁₋₆alkylheterocyclyl are optionally substituted with 1 to 3 groups selected from halo, C₁₋₆alkyl, C₁₋₆haloalkyl, C₁₋₆alkoxy, C₁₋₆haloalkoxy, -N(R^{e})₂, -C(O)R^{f}, and -C₁₋₆alkylOR^{e};
each R^{e} is independently hydrogen, halo(C₁₋₄alkyl), or C₁₋₄alkyl;
each R^{f} is independently hydrogen, halo(C₁₋₄alkyl), C₁₋₄alkyl, or
(C₃-C₄)cycloalkyl;
q is 0 or 1; and
p is 1,
wherein:
the term aryl refers to an aromatic carbocyclic single ring or two fused ring system containing 6 to 10 carbon atoms;
the term heteroaryl used alone or as part of a larger moiety refers to a 5- to 12-membered aromatic radical containing 1 to 4 heteroatoms selected from N, O, and S; and
the term heterocyclyl refers to a 4- to 12-membered saturated or partially unsaturated heterocyclic ring containing 1 to 4 heteroatoms independently selected from N, O, and S.

2. The compound of Claim 1 wherein the compound is of the Formula **V:** 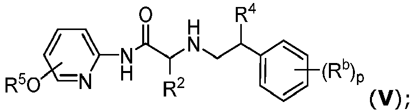 or a pharmaceutically acceptable salt thereof.

3. The compound of Claim 1 or 2, wherein the compound is of the Formula **VI** or **VII:** 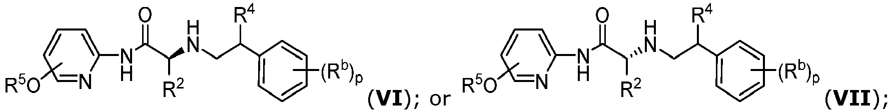 or a pharmaceutically acceptable salt thereof.

4. The compound of any one of Claims 1 to 3, wherein the compound is of the Formula **VIII, IX, X,** or **XI:** 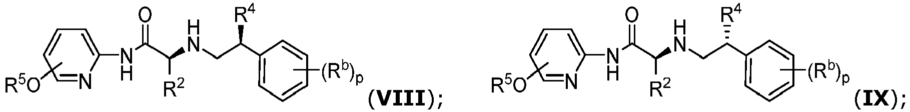 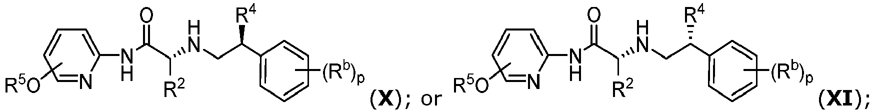 or a pharmaceutically acceptable salt thereof.

5. The compound of any one of Claims 1 to 4, wherein R² is phenyl.

6. The compound of any one of Claims 1 to 5, wherein R^{b} is cyano.

7. The compound of any one of Claims 1 to 6, wherein R⁵ is a C₁₋₄alkyl substituted with pyrazolyl or oxazolidinyl, each of which are optionally substituted with 1 to 3 groups selected from R^{d}; or R⁵ is piperidinyl, azetidinyl, hexahydropyrimidinyl, tetrahydrofuranyl, tetrahydropyranyl, oxetanyl, pyrazolyl, pyrrolidinyl, or pyrimidinyl, each of which are optionally substituted with 1 to 3 groups selected from R^{d}.

8. The compound of any one of Claims 1 to 7, wherein R^{d} is selected from halo, oxo, C₁₋₄alkyl, C₁₋₄alkoxy, halo(C₁₋₄alkyl), -C₁₋₄alkylOR^{e}, -C(O)R^{f}, -C(O)N(R^{e})₂, -C₁₋₆alkylC(O)N(R^{e})₂, and -S(O)₂R^{e}.

9. The compound of any one of Claims 1 to 8, wherein R^{d} is selected from C₁₋₆alkyl, -C(O)R^{f}, and oxo.

10. The compound of any one of Claims 1 to 8, wherein R^{e} is hydrogen or C₁₋₃alkyl.

11. The compound of any one of Claims 1 to 10, wherein R^{f} is cyclopropyl or C₁₋₄alkyl.

12. The compound of any one of Claims 1 to 11, wherein R⁵ is

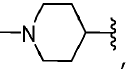

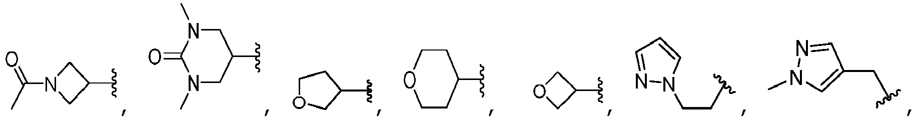

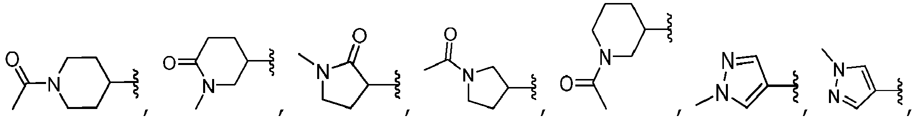

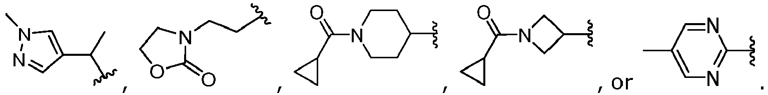

13. The compound of Claim 1, wherein the compound is selected from 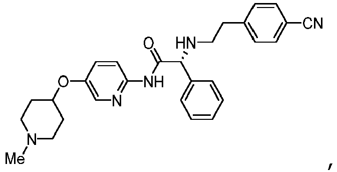 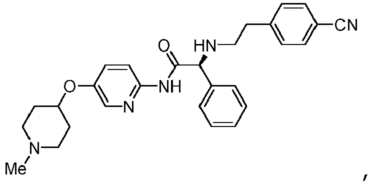 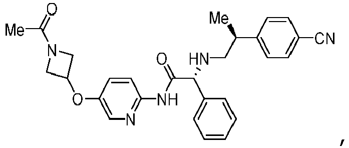 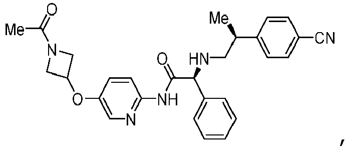 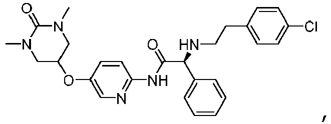 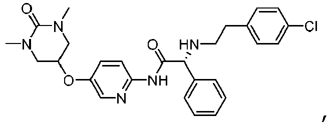 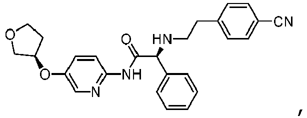 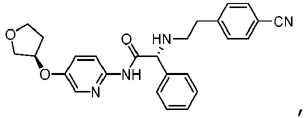 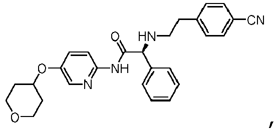 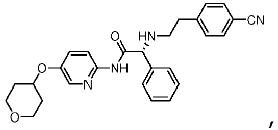 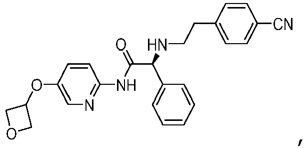 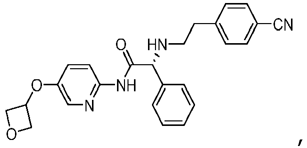 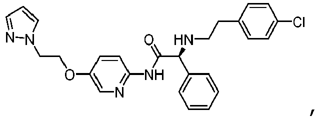 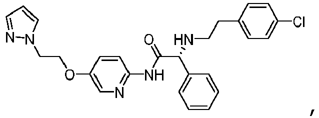 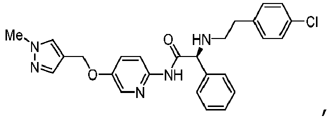 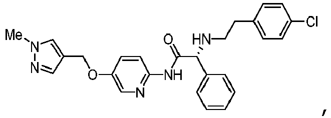 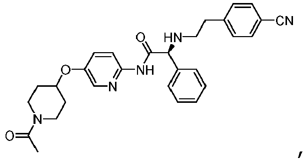 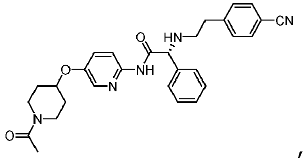 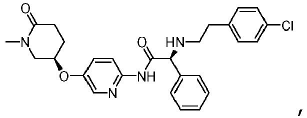 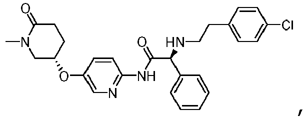 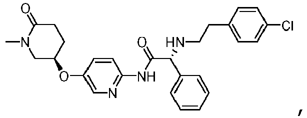 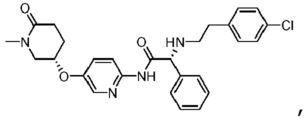 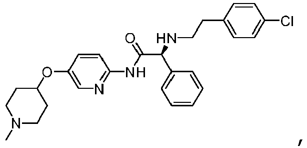 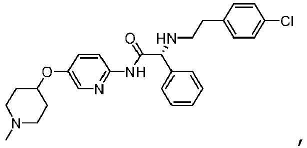 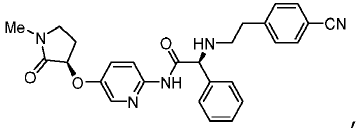 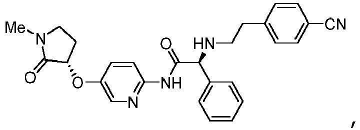 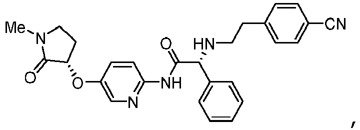 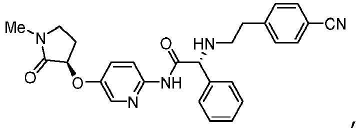 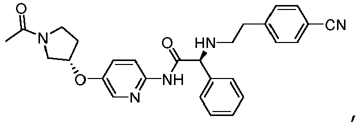 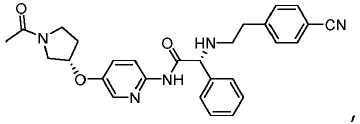 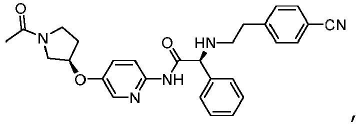 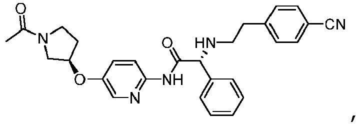 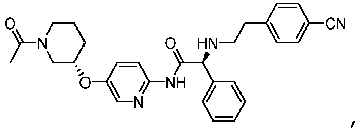 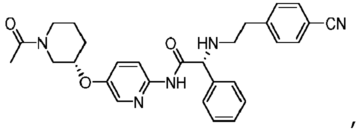 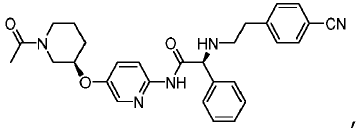 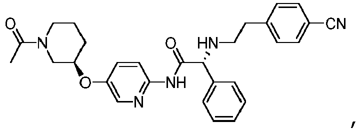 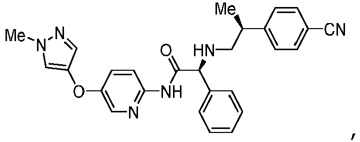 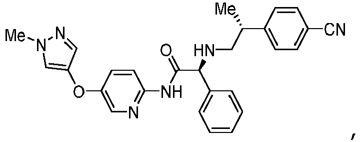 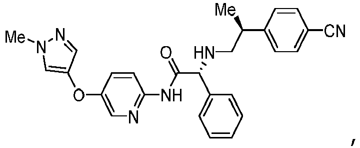 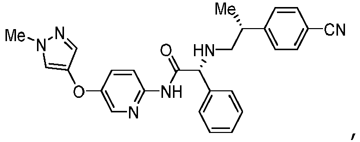 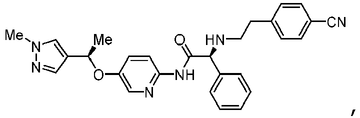 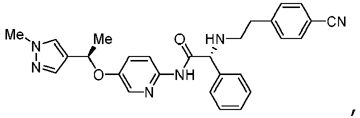 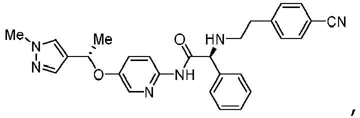 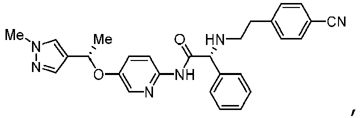 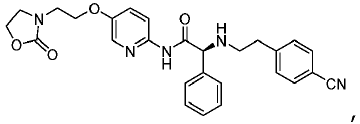 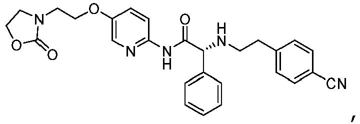 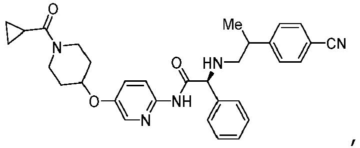 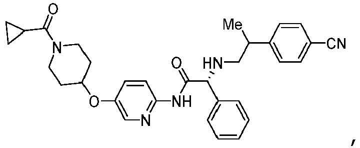 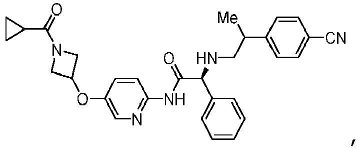 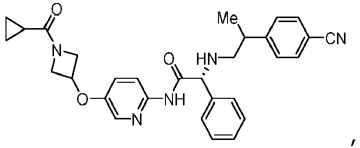 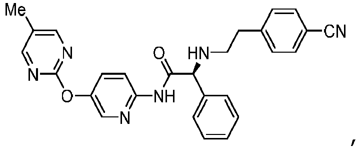 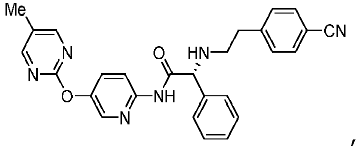 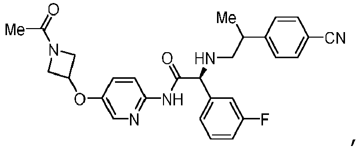 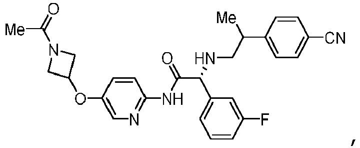 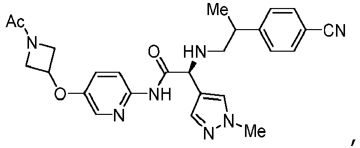 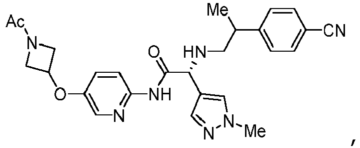 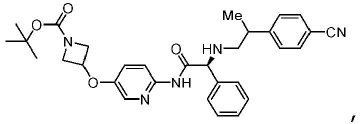 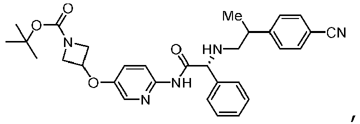 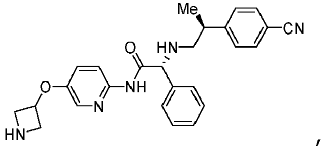 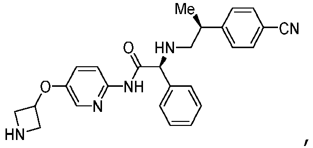 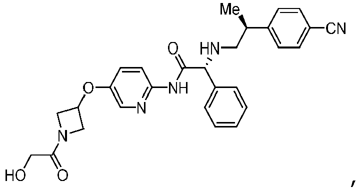 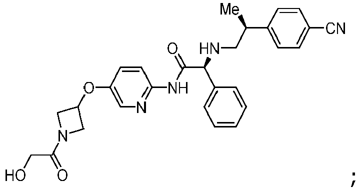 or a pharmaceutically acceptable salt thereof.

14. A pharmaceutical composition comprising a compound of any one of Claims 1 to 13, or a pharmaceutically acceptable salt thereof; and a pharmaceutically acceptable carrier.

15. The compound of any one of Claims 1 to 13, or a pharmaceutically acceptable salt thereof, or the composition of Claim 14, for use in treating a CBP and/or EP300-mediated disorder selected from a cancer, a cardiac disease, a metabolic disease, a fibrotic disease, an inflammatory disease, and a viral infection.

## Patentansprüche

1. Verbindung mit der Formel **I:** 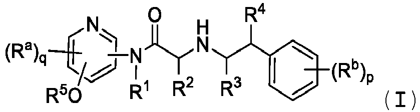 oder ein pharmazeutisch annehmbares Salz davon, wobei
R¹, R³ und R⁴ jeweils unabhängig Wasserstoff oder C₁₋₄Alkyl sind;
R² Phenyl oder Pyrazolyl ist, welche jeweils optional durch Halo oder C₁₋₄Alkyl substituiert sind;
R⁵ ein mit 4- bis 6-gliedrigem Heterocyclyl oder 5-bis 6-gliedrigem Heteroaryl substituiertes C₁₋₆Alkyl ist, wobei das 4- bis 6-gliedrige Heterocyclyl oder 5- bis 6-gliedrige Heteroaryl jeweils optional durch 1 bis 3 Gruppen substituiert sind, welche ausgewählt sind aus R^{d}; oder R⁵ 4- bis 6-gliedriges Heterocyclyl oder 5- bis 6-gliedriges Heteroaryl ist, wobei das 4- bis 6-gliedrige Heterocyclyl oder 5- bis 6-gliedrige Heteroaryl jeweils optional durch 1 bis 3 Gruppen, ausgewählt aus R^{d} substituiert sind;
R^{a}, falls vorhanden, C₁₋₃Alkyl, C₁₋₃Alkoxy, Halo(C₁₋₃Alkyl), HaloC₁₋₃Alkoxy oder Halo ist;
R^{b} Halo, Cyan oder -SO₂NH₂ ist;
R^{d} jeweils unabhängig Halo, CN, Oxo, NO₂, C₁₋₆Alkyl, C₂₋₆Alkenyl, C₁₋₆Alkoxy, Halo(C₁₋₆Alkoxy), Halo(C₁₋₆Alkyl), - C₁₋₆AlkylOR^{e}, -C(O)R^{f}, -C(O)OR, -C₁₋₆AlkylC(O)OR^{e}, - C(O)N(R^{e})₂, -C(O)NR^{e}C₁₋₆AlkylOR^{e}, -OC₁₋₆AlkylN(R^{e})₂, -C₁₋₆AlkylC(O)N(R^{e})₂, -C₁₋₆AlkylN(R^{e})₂, -N(R^{e})₂, -C(O)NR^{e}C₁₋₆AlkylN(R^{e})₂, -NR^{e}C₁₋₆AlkylN(R^{e})₂, -NR^{e}C₁₋₆AlkylOR^{e}, - SOR^{e}, -S(O)₂R^{e}, -SON(R^{e})₂, -SO₂N(R^{e})₂, -O(C₃-C₆)Cycloalkyl, -O-C₁₋₄Alkylaryl, -C₁₋₆Alkyl(C₃-C₆)Cycloalkyl, -C₁₋₆Alkylaryl, -C₁₋₆Alkylheteroaryl, -C₁₋₆Alkylheterocyclyl, (C₃-C₆)Cycloalkyl, Heterocyclyl, Heteroaryl oder Aryl ist, wobei das (C₃-C₆)Cycloalkyl, Heterocyclyl, Aryl und Heteroaryl jeweils allein und in Verbindung mit -O(C₃₋C₆) Cycloalkyl, -C₁₋₆Alkyl(C₃-C₆)Cycloalkyl, -C₁₋₆Alkylaryl, -C₁₋₆Alkylheteroaryl, und -C₁₋₆Alkylheterocyclyl optional durch 1 bis 3 Gruppen, ausgewählt aus Halo, C₁₋₆Alkyl, C₁₋₆Haloalkyl, C₁₋₆Alkoxy, C₁₋₆Haloalkoxy, -N(R^{e})₂, -C(O)R^{f} und -C₁₋₆AlkylOR^{e} substituiert sind;
das R^{e} jeweils unabhängig Wasserstoff, Halo (C₁₋₄Alkyl) oder C₁₋₄Alkyl ist;
das R^{f} jeweils unabhängig Wasserstoff, Halo (C₁₋₄Alkyl), C₁₋₄Alkyl oder
(C₃-C₄) Cycloalkyl ist;
q 0 oder 1 ist; und
p 1 ist;
wobei:
der Begriff Aryl sich auf einen aromatischen carbocyclischen Einzelring oder zwei kondensierte Ringsysteme bezieht, welche 6 bis 10 Kohlenstoffatome enthalten;
der Begriff Heteroaryl allein oder als Teil eines größeren Teils verwendet sich auf ein 5- bis 12-gliedriges aromatisches Radikal bezieht, welches 1 bis 4 Heteroatome, ausgewählt aus N, O und S enthält; und
der Begriff Heterocyclyl sich auf einen 4- bis 12-gliedrigen gesättigten oder teilweise ungesättigten heterocyclischen Ring bezieht, welcher 1 bis 4 Heteroatome, unabhängig ausgewählt aus N, O und S enthält.

2. Verbindung nach Anspruch 1, wobei die Verbindung der Formel V entspricht: 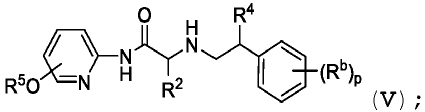 oder ein pharmazeutisch annehmbares Salz davon.

3. Verbindung nach Anspruch 1 oder 2, wobei die Verbindung der Formel **VI** oder **VII** entspricht: 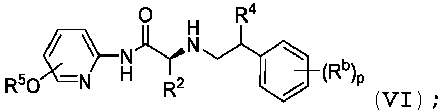 oder 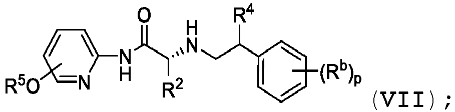 oder ein pharmazeutisch annehmbares Salz davon.

4. Verbindung nach einem der Ansprüche 1 bis 3, wobei die Verbindung der Formel **VIII, IX, X** oder **XI** entspricht: 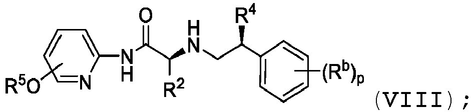 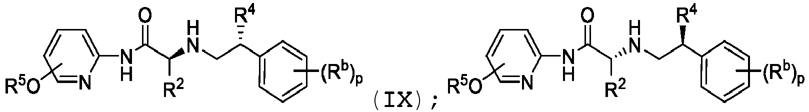 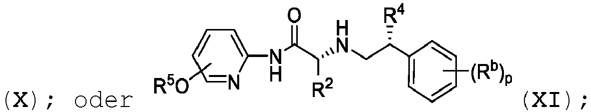 oder ein pharmazeutisch annehmbares Salz davon.

5. Verbindung nach einem der Ansprüche 1 bis 4, wobei R² Phenyl ist.

6. Verbindung nach einem der Ansprüche 1 bis 5, wobei R^{b} Cyan ist.

7. Verbindung nach einem der Ansprüche 1 bis 6, wobei R⁵ ein durch Pyrazolyl oder Oxazolidinyl substituiertes C₁₋₄Alkyl ist, welche jeweils optional durch 1 bis 3 Gruppen, ausgewählt aus R^{d} substituiert sind; oder R⁵ Piperidinyl, Azetidinyl, Hexahydropyrimidinyl, Tetrahydrofuranyl, Tetrahydropyranyl, Oxetanyl, Pyrazolyl, Pyrrolidinyl oder Pyrimidinyl ist, welche jeweils optional durch 1 bis 3 Gruppen, ausgewählt aus R^{d} substituiert sind.

8. Verbindung nach einem der Ansprüche 1 bis 7, wobei R^{d} aus Halo, Oxo, C₁₋₄Alkyl, C₁₋₄Alkoxy, Halo (C₁₋₄Alkyl), - C₁₋₄AlkylOR^{e}, -C(O)R^{f}, -C(O)N(R^{e})₂, -C₁₋₆AlkylC(O)N(R^{e})₂ und -S(O)₂R^{e} ausgewählt ist.

9. Verbindung nach einem der Ansprüche 1 bis 8, wobei R^{d} aus C₁₋₆Alkyl, -C(O)R^{f} und Oxo ausgewählt ist.

10. Verbindung nach einem der Ansprüche 1 bis 8, wobei R^{e} Wasserstoff oder C₁₋₃Alkyl ist.

11. Verbindung nach einem der Ansprüche 1 bis 10, wobei R^{f} Cyclopropyl oder C₁₋₄Alkyl ist.

12. Verbindung nach einem der Ansprüche 1 bis 11, wobei R5 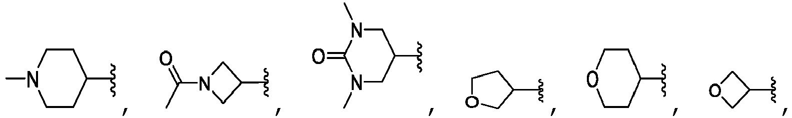 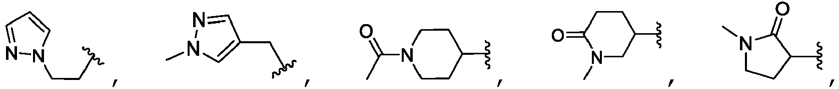 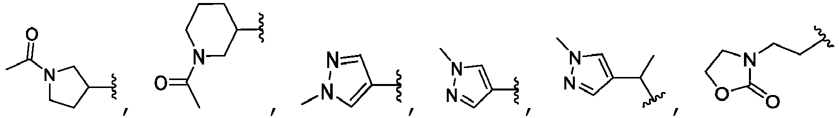 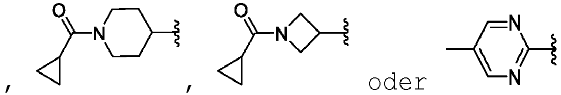 ist.

13. Verbindung nach Anspruch 1, wobei die Verbindung aus 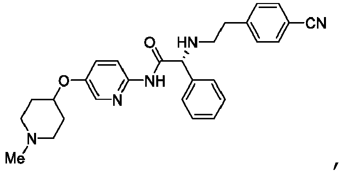 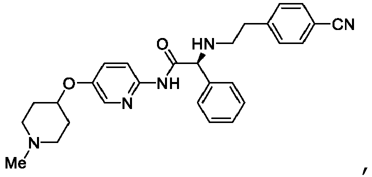 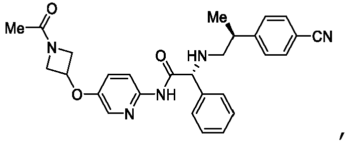 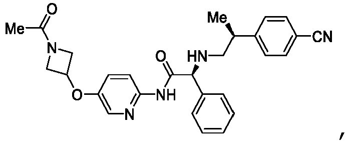 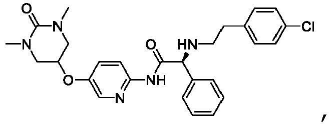 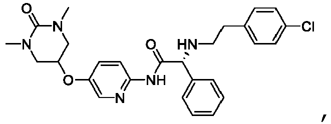 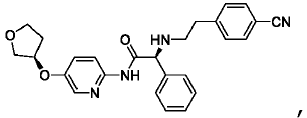 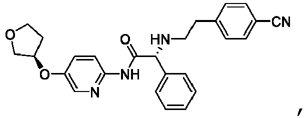 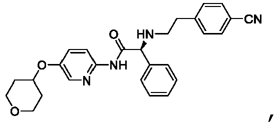 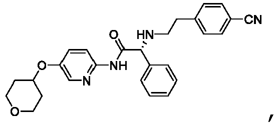 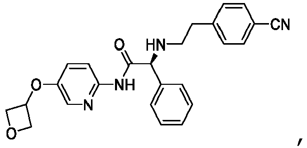 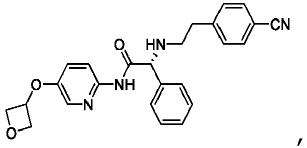 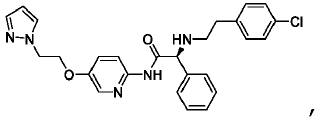 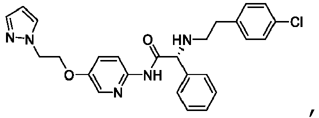 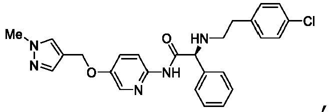 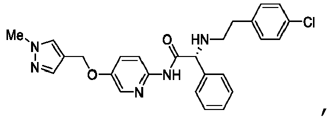 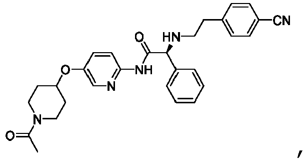 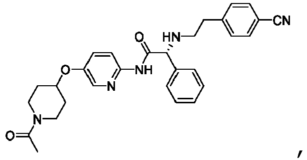 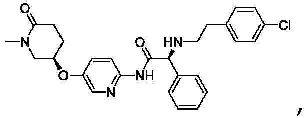 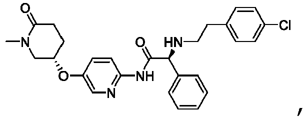 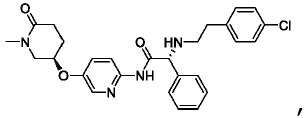 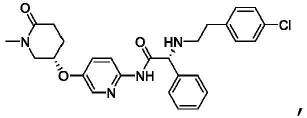 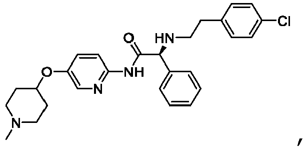 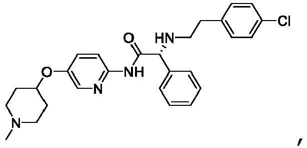 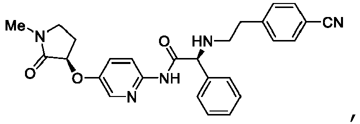 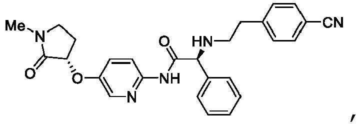 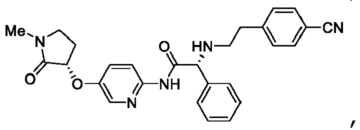 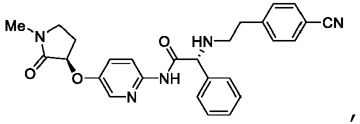 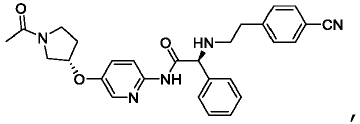 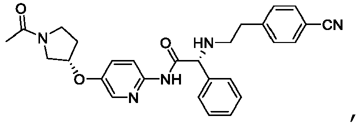 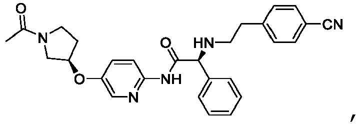 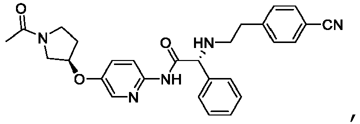 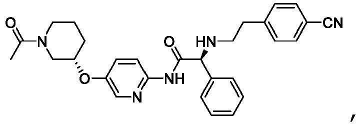 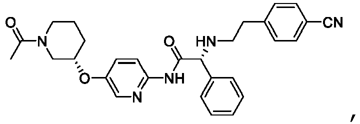 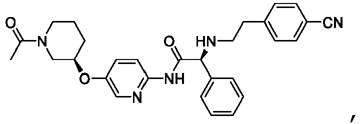 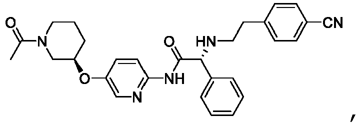 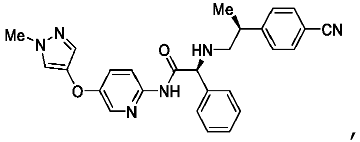 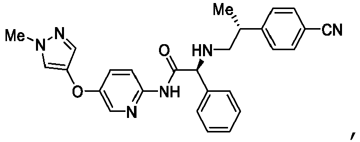 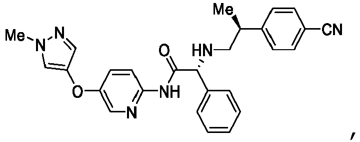 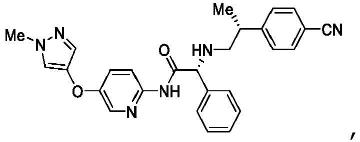 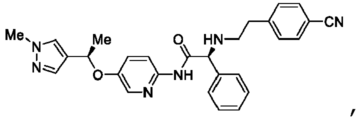 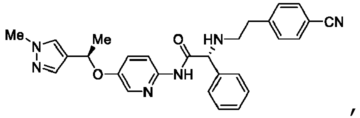 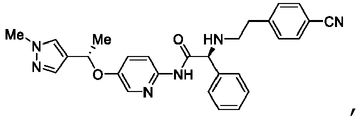 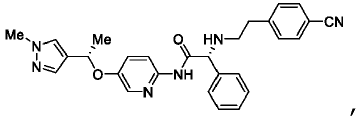 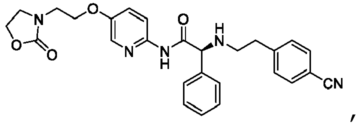 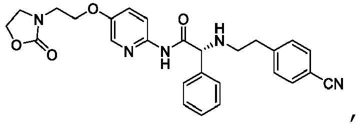 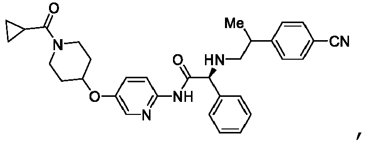 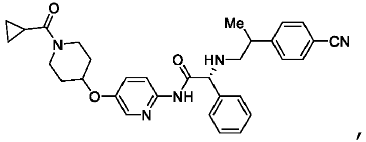 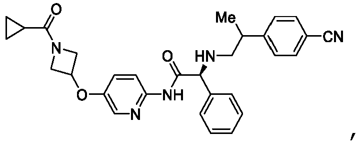 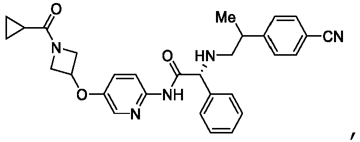 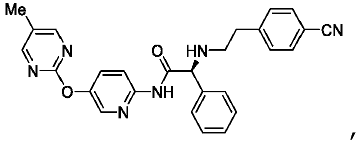 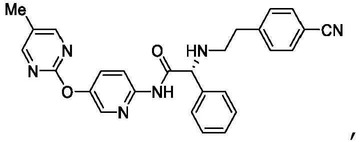 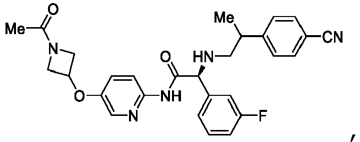 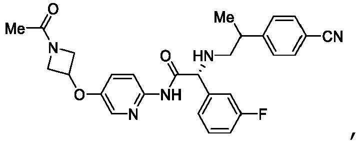 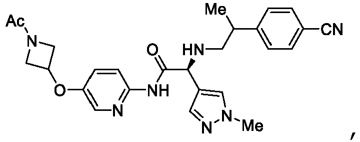 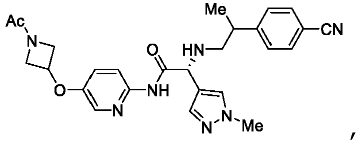 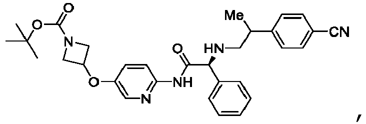 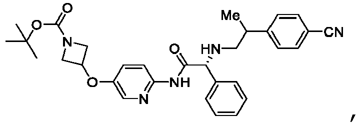 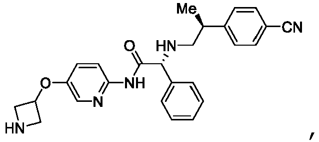 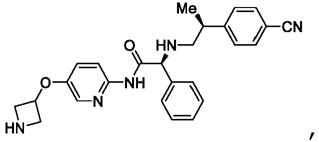 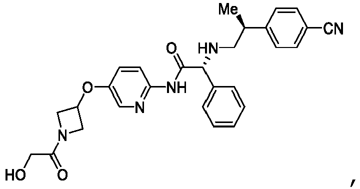 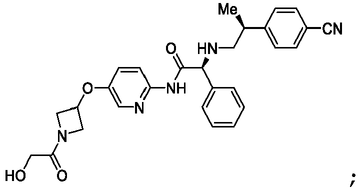 oder einem pharmazeutisch annehmbaren Salz davon ausgewählt ist.

14. Pharmazeutische Zusammensetzung, umfassend eine Verbindung nach einem der Ansprüche 1 bis 13, oder ein pharmazeutisch annehmbares Salz davon; und eine pharmazeutisch annehmbare Trägersubstanz.

15. Verbindung nach einem der Ansprüche 1 bis 13, oder ein pharmazeutisch annehmbares Salz davon, oder die Zusammensetzung nach Anspruch 14 zur Verwendung in der Behandlung einer CBP- und/oder EP300-vermittelten Störung, ausgewählt aus einem Krebs, einer Herzerkrankung, einer Stoffwechselerkrankung, einer fibrotischen Erkrankung, einer Entzündungskrankheit und einer Virusinfektion.

## Revendications

1. Composé répondant à la Formule **I** : 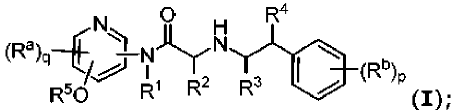 ou un sel pharmaceutiquement acceptable de celui-ci ; dans lequel
R¹, R³ et R⁴ sont chacun indépendamment un hydrogène ou un alkyle en C₁₋₄ ;
R² est un phényle ou un pyrazolyle, chacun étant éventuellement substitué par un halogéno ou un alkyle en C₁₋₄ ;
R⁵ est un alkyle en C₁₋₆ substitué par un hétérocyclyle à 4- à 6- chaînons ou un hétéroaryle à 5-à 6- chaînons, dans lequel chacun desdits hétérocyclyle à 4- à 6- chaînons ou hétéroaryle à 5- à 6- chaînons est éventuellement substitué par 1 à 3 groupes choisis parmi R^{d} ; ou R⁵ est un hétérocyclyle à 4- à 6 chaînons ou un hétéroaryle à 5- à 6 chaînons, dans lequel chacun desdits hétérocyclyle à 4- à 6 chaînons ou hétéroaryle à 5- à 6 chaînons est éventuellement substitué par 1 à 3 groupes choisis parmi R^{d} ;
R^{a}, s'il est présent, est un alkyle en C₁₋₃, un alcoxy en C₁₋₃, un halogénoalkyle en C₁₋₃, un halogénoalcoxy en C₁₋₃ ou un halogéno ;
R^{b} est un halogène, un cyano ou -SO₂NH₂ ;
chaque R^{d} est indépendamment un halogène, CN, oxo, NO₂, alkyle en C₁₋₆, alcényle en C₂₋₆, alcoxy en C₁₋₆, halogénoalcoxy en C₁₋₆, halogénoalkyle en C₁₋₆, - (alkyl en C₁₋₆)OR^{e}, -C(O)R^{f}, -C(O)OR, -(alkyl en C₁₋₆)C(O)OR^{e}, - C(O)N(R^{e})₂, -C(O)NR^{e}(alkyl en C₁₋₆)OR^{e}, -O (alkyl en C₁₋₆)N(R^{e})₂, - (alkyl en C₁₋₆)C(O)N(R^{e})₂, - (alkyl en C₁₋₆)N(R^{e})₂, -N(R^{e})₂, -C(O)NR^{e}(alkyl en C₁₋₆)N(R^{e})₂, -NR^{e}(alkyl en C₁₋₆)N(R^{e})₂, -NR^{e}(alkyl en C₁₋₆)OR^{e}, -SOR^{e}, -S(O)₂R^{e}, -SON(R^{e})₂, -SO₂N(R^{e})₂, -Ocycloalkyle en C₃-C₆, - O-(alkyl en C₁₋₄)aryle, - (alkyl en C₁₋₆)cycloalkyle en C₃-C₆, - (alkyl en C₁₋₆)aryle, - (alkyl en C₁₋₆)hétéroaryle, - (alkyl en C₁₋₆)hétérocyclyle, cycloalkyle en C₃-C₆, hétérocyclyle, hétéroaryle ou aryle, dans lequel chacun desdits cycloalkyle en C₃-C₆, hétérocyclyle, aryle et hétéroaryle seul et en relation avec -Ocycloalkyle en C₃-C₆, - (alkyl en C₁₋₆)cycloalkyle en C₃₋-C₆, - (alkyl en C₁₋₆)aryle, - (alkyl en C₁₋₆)hétéroaryle, et - (alkyl en C₁₋₆)hétérocyclyle est éventuellement substitué par 1 à 3 groupes choisis parmi halogène, alkyle en C₁₋₆, halogénoalkyle en C₁₋₆, alkoxy en C₁₋₆, halogénoalkoxy en C₁₋₆, -N(R^{e})₂, -C(O)R^{f} et - (alkyl en C₁₋₆)OR^{e} ;
chaque R^{e} est indépendamment un hydrogène, un halogénoalkyle en C₁₋₄ ou un alkyle en C₁₋₄ ;
chaque R^{f} est indépendamment un hydrogène, un halogénoalkyle en C₁₋₄, un alkyle en C₁₋₄ ou un cycloalkyle en C₃-C₄ ;
q vaut 0 ou 1 ; et
p vaut 1,
dans lequel :
le terme aryle fait référence à un cycle carbocyclique aromatique unique ou à un système à deux cycles condensés contenant 6 à 10 atomes de carbone ;
le terme hétéroaryle utilisé seul ou en tant que partie d'un groupement plus grand fait référence à un radical aromatique de 5- à 12 chaînons contenant 1 à 4 hétéroatomes choisis parmi N, O et S ; et
le terme hétérocyclyle fait référence à un cycle hétérocyclique saturé ou partiellement insaturé de 4- à 12 chaînons contenant 1 à 4 hétéroatomes indépendamment choisis parmi N, O et S.

2. Composé selon la revendication 1, dans lequel le composé répond à la formule V : 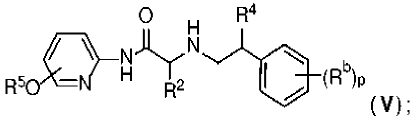 ou un sel pharmaceutiquement acceptable de celui-ci.

3. Composé selon la revendication 1 ou 2, dans lequel le composé répond à la formule VI ou VII : 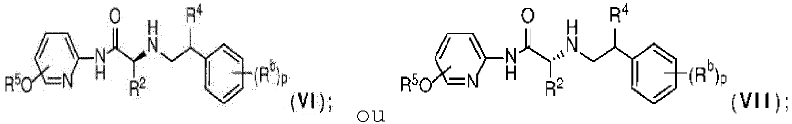 ou un sel pharmaceutiquement acceptable de celui-ci.

4. Composé selon l'une quelconque des revendications 1 à 3, dans lequel le composé répond à la formule **VIII, IX, X** ou **XI** : 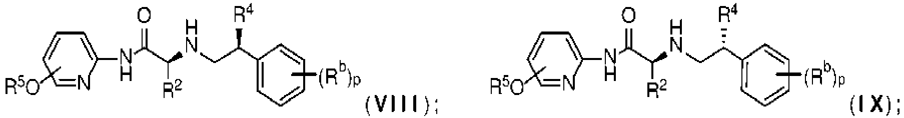 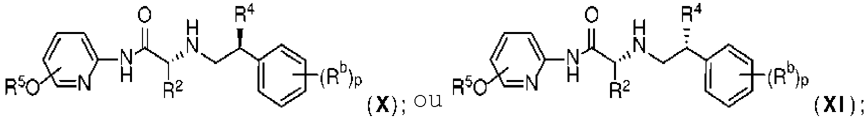 ou un sel pharmaceutiquement acceptable de celui-ci.

5. Composé selon l'une quelconque des revendications 1 à 4, dans lequel R² est un phényle.

6. Composé selon l'une quelconque des revendications 1 à 5, dans lequel R^{b} est un cyano.

7. Composé selon l'une quelconque des revendications 1 à 6, dans lequel R⁵ est un alkyle en C₁₋₄ substitué par un pyrazolyle ou un oxazolidinyle, chacun étant éventuellement substitué par 1 à 3 groupes choisis parmi R^{d} ; ou R⁵ est un pipéridinyle, azétidinyle, hexahydropyrimidinyle, tétrahydrofuranyle, tétrahydropyrannyle, oxétanyle, pyrazolyle, pyrrolidinyle ou pyrimidinyle, chacun étant éventuellement substitué par 1 à 3 groupes choisis parmi R^{d}.

8. Composé selon l'une quelconque des revendications 1 à 7, dans lequel R^{d} est choisi parmi un halogéno, oxo, alkyle en C₁₋₄, alcoxy en C₁₋₄, halogénoalkyle en C₁₋₄, -(alkyl en C₁₋₄)OR^{e}, -C(O)R^{f}, - C(O)N(R^{e})₂, -(alkyl en C₁₋₆)C(O)N(R^{e})₂ et -S(O)₂R^{e}.

9. Composé selon l'une quelconque des revendications 1 à 8, dans lequel R^{d} est choisi parmi un alkyle en C₁₋₆, -C(O)R^{f} et oxo.

10. Composé selon l'une quelconque des revendications 1 à 8, dans lequel R^{e} est un hydrogène ou un alkyle en C₁₋₃.

11. Composé selon l'une quelconque des revendications 1 à 10, dans lequel R^{f} est un cyclopropyle ou un alkyle en C₁₋₄.

12. Composé selon l'une quelconque des revendications 1 à 11, dans lequel R⁵ est

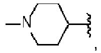

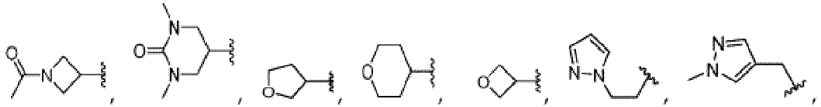

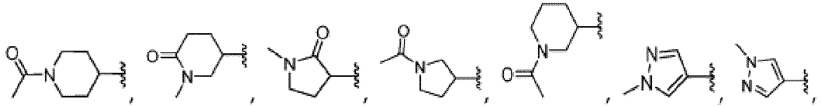

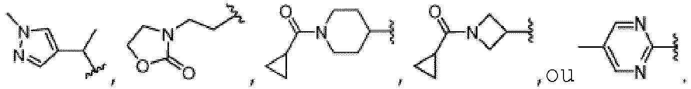

13. Composé selon la revendication 1, dans lequel le composé est choisi parmi 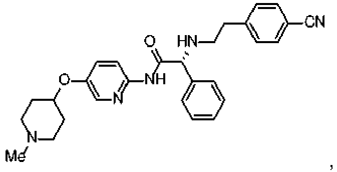 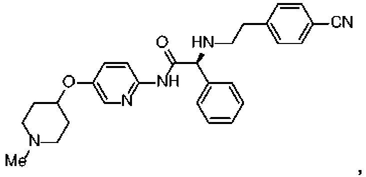 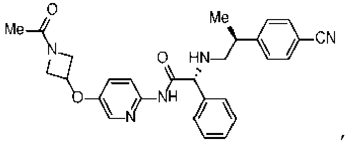 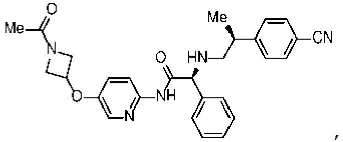 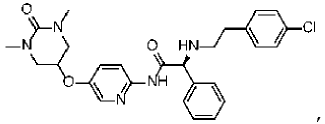 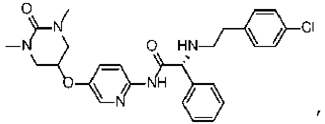 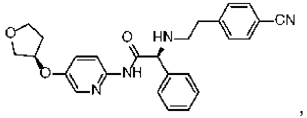 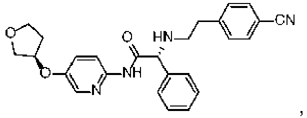 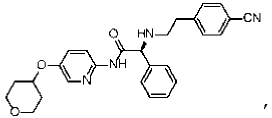 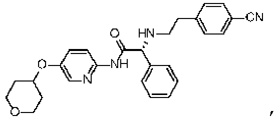 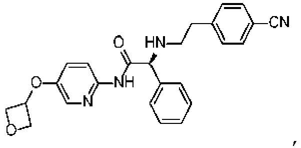 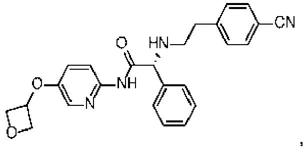 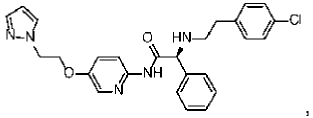 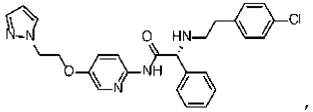 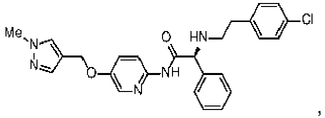 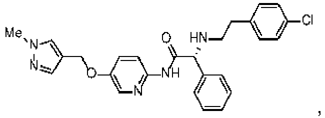 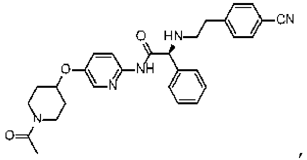 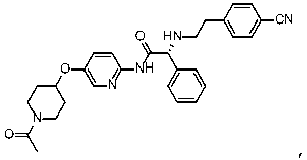 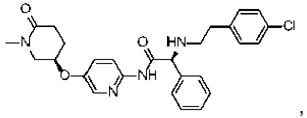 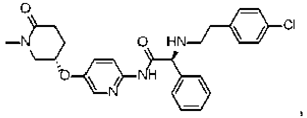 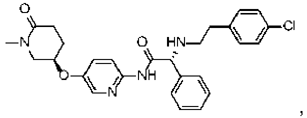 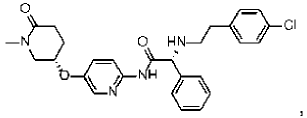 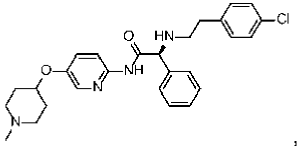 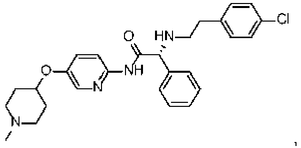 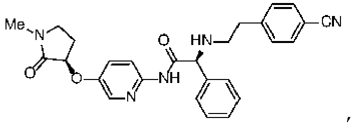 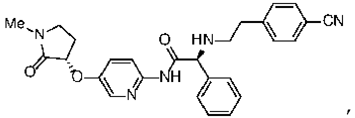 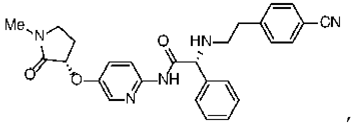 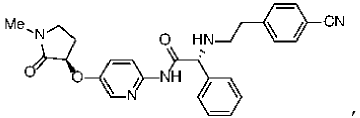 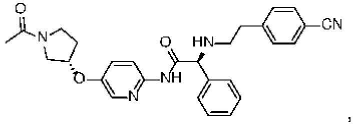 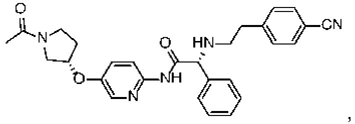 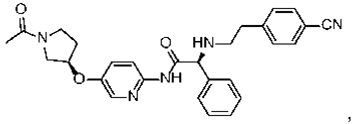 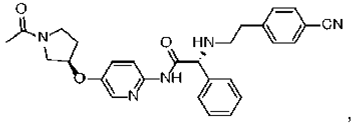 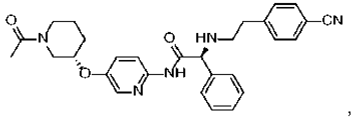 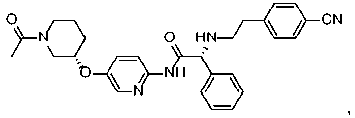 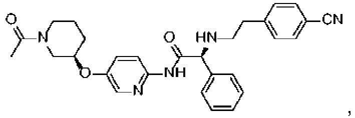 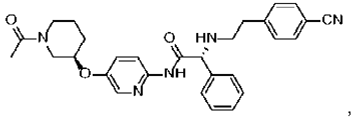 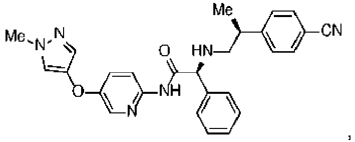 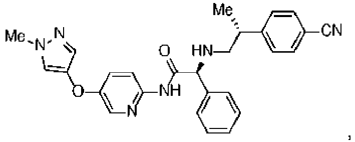 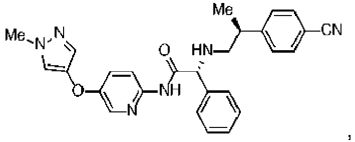 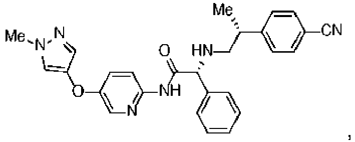 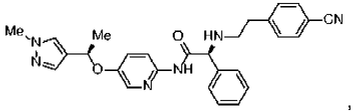 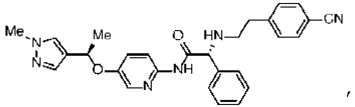 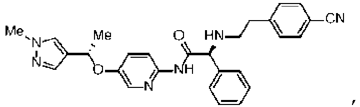 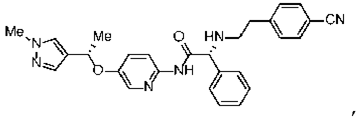 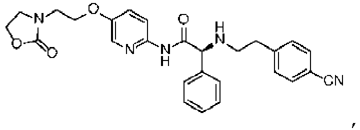 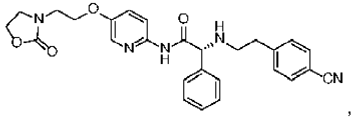 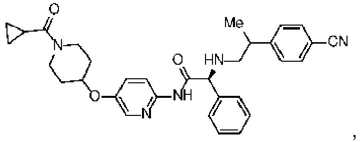 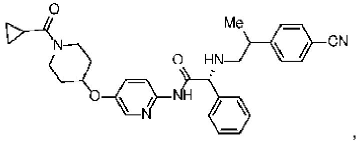 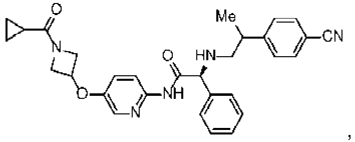 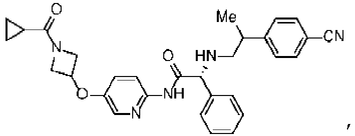 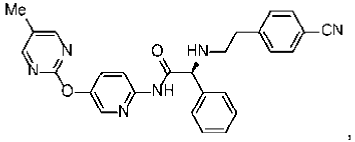 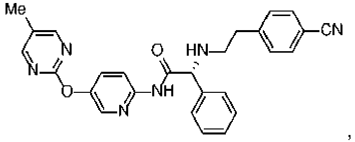 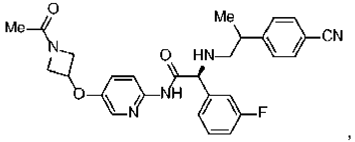 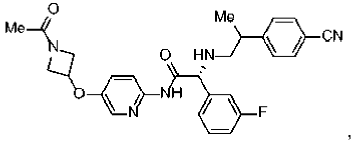 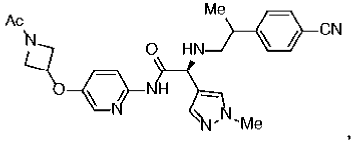 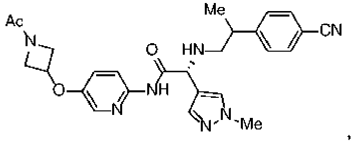 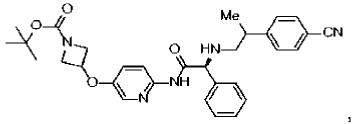 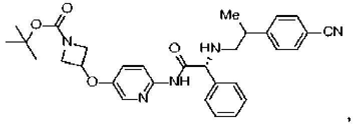 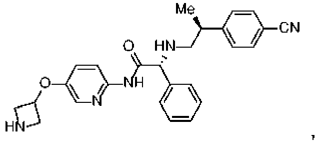 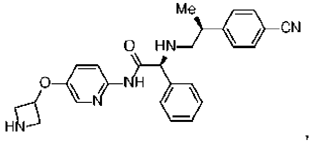 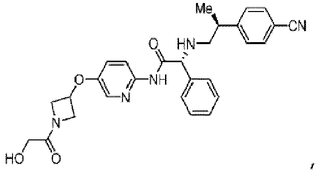 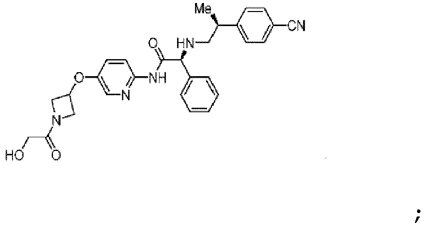 ou un sel pharmaceutiquement acceptable de celui-ci.

14. Composition pharmaceutique comprenant un composé selon l'une quelconque des revendications 1 à 13, ou un sel pharmaceutiquement acceptable de celui-ci ; et un entraîneur pharmaceutiquement acceptable.

15. Composé selon l'une quelconque des revendications 1 à 13, ou sel pharmaceutiquement acceptable de celui-ci, ou composition selon la revendication 14, destiné à être utilisé dans le traitement d'un trouble médié par le CBP et/ou l'EP300-choisi parmi un cancer, une maladie cardiaque, une maladie métabolique, une maladie fibrotique, une maladie inflammatoire et une infection virale.
